# EUROPÄISCHE PATENTSCHRIFT

(11) **EP 1 292 317 B1**
(45) Veröffentlichungstag und Bekanntmachung des Hinweises auf die Patenterteilung: **10.08.2005**
(21) Anmeldenummer: 01945182.2
(22) Anmeldetag: 22.05.2001
(51) Int. Cl.: A61K 35/23, A61P 9/02, A61P 9/12

(54) **BLUTDRUCKSENKENDE UND BLUTDRUCKERHÖHENDE AKTIVITÄTEN AUS DEM NIERENMARK**
HYPOTENSIVE AND HYPERTENSIVE PROPERTIES OF RENOMEDULLAR EXTRACTS
EXTRAITS DE LA SUBSTANCE MEDULLAIRE RENALE AYANT UNE ACTIVITE D'ABAISSEMENT OU D'ELEVATION DE LA PRESSION SANGUINE

(30) Priorität: 26.05.2000 EP 00111350; 21.11.2000 DE 10057913
(43) Veröffentlichungstag der Anmeldung: 19.03.2003
(73) Patentinhaber: Angion GmbH & Co. KG, 49076 Osnabrück (DE)
(72) Erfinder: GLODNY, Bernhard, 48161 Münster (DE)
(74) Vertreter: Weber, Thomas, Dr.Dipl.-Chem.
(86) Internationale Anmeldenummer: PCT/EP2001/005872
(87) Internationale Veröffentlichungsnummer: WO 2001/091776

(56) Entgegenhaltungen:
- US-A- 3 683 070
- MUIRHEAD E E ET AL: "Derivation of antihypertensive neutral renomedullary lipid from renal venous effluent." JOURNAL OF LABORATORY AND CLINICAL MEDICINE, (1982 JAN) 99 (1) 64-75., XP000940949 in der Anmeldung erwähnt
- SHIMIZU K ET AL: "Identification of vasopressin and determination of its corticomedullary levels in rat kidney tissue." KIDNEY INTERNATIONAL, (1984 DEC) 26 (6) 785-90., XP000941083
- MUIRHEAD, E. ERIC ET AL: "Cardiovascular effects of antihypertensive polar and neutral renomedullary lipids" HYPERTENSION (DALLAS) (1983), 5(2, PT. 2), 112-18, XP000941013

## Beschreibung

Die Erfindung betrifft neue, den Blutdruck senkende und den Blutdruck erhöhende Extrakte, die aus dem Nierenmark erhältlich sind, Verfahren zu deren Isolierung aus dem Nierenmark von Säugetieren, pharmazeutische Zusammensetzungen umfassend die jeweiligen Extrakte sowie deren Verwendung zur Senkung und Erhöhung des Blutdruckes bei Säugetieren, insbesondere beim Menschen.

### Hintergrund der Erfindung

Die Niere enthält eine Reihe von Substanzen, die blutdruckwirksam sind. Diese Substanzen lassen sich einteilen in blutdrucksenkende, wie das Prostaglandin E2 oder Prostaglandin A2, das artifizielle Aglykon, und blutdrucksteigernde Substanzen, wie das Thromboxan A2. Die meisten der Substanzen, wie z. B. Nephrotensin, Renotensin, Corticopressin oder Medullipin, sind bis heute nicht rein gewonnen worden und in Ihrer Struktur unbekannt. Auch das Thromboxan A2 ist aus der Niere nicht rein gewonnen worden.

Daher erregte es große Aufmerksamkeit, als 1941 von Page et al. (Page I.H., Helmer O.M., Kohlstaedt K.G., Gambill W.D., Taylor R.D. in "The blood pressure reducing property of extracts of kidneys in hypertensive patients and animals" Ann. Int. Med. 15, 347 (1941)) berichtet wurde, dass mit Nierenextrakten Augenhintergrundsveränderungen von an maligner Hypertonie erkrankten Patienten zurückgebildet, und hoher Blutdruck gesenkt werden konnte. Mit Experimenten an Hunden, in denen eine renoprive Hypertonie mit der intraperitonealen Gabe von Nierenmark, nicht aber von Nierenrinde gebessert werden konnte (Muirhead E.E., Jones F., Stirman J.A. in "Antihypertensive property in renoprival hypertension of extracts of renal medulla" J. Lab. Clin. Med. 56, 167 (1960)) wurde klar, dass das den Blutdruck senkende Prinzip im Nierenmark enthalten sein musste. Dies führte schließlich zur Isolierung der stark vasodilatorisch wirkenden Prostaglandine A2 und E2 aus Nierenmark (Daniels E.G., Hinman J.W., Leach B.E., Muirhead E.E. in "Identification of prostaglandin E2 as the principle vasodepressor lipid of rabbit renal medulla" Nature 215, 1298 (1967)). Das Prostaglandin E2 wurde als das hauptsächlich wirksame blutdrucksenkende Prinzip des Nierenmarkes beschrieben (Daniels, o.a.O.).

Das später in *Medullipin* umbenannte *Antihypertensive Neutral Renomedullary Lipid* (ANRL) (Muirhead E.E. in "Renomedullary vasodepressor Lipid: Medullipin" in "Textbook of hypertension" Herausgeber: J. D. Swales. Oxford, Blackwell, 1994, nachfolgend "Textbook" genannt) konnte bis jetzt nicht aufgereinigt werden (Muirhead E.E. in "Textbook" o.a.O.)).

Die stoffchemische Zuordnungen verschiedener beobachteter physiologischer Phänomene zu einem Molekül Medullipin (Muirhead, E.E., "Textbook" o.a.O.) sind bis heute hypothetisch. So gibt es bis heute lediglich die chemisch unbewiesene Vermutung, Medullipin besitze zwei einander benachbarte Hydroxylgruppen (Muirhead E.E., Byers L.W., Brooks B., Brown P., Pitcock J.A. in "Biologic contrasts between Medullipin I and vasoactive glyceryl compounds", Am. J. Med. Sci. 1989; 298: 93-103). Medullipin ist bis heute nur physiologisch charakterisiert:
*Bezüglich der Verbreitung bei den Mammalia:* Medullipin ist im Nierenmark des Menschen, des Kaninchen und der Ratte enthalten (Muirhead E.E., in "Textbook" o.a.O.).
*Bezüglich der Gewinnung:* Es kann aus Plasma aus der Vene einer Niere gewonnen werden, die mit erhöhtem Perfusionsdruck durchflossen wird. Solches Plasma hat einen vom Perfusionsdruck direkt abhängigen blutdrucksenkenden Effekt an spontan hypertensiven Ratten. Es kann darüber hinaus aus frischem Nierenmark, wie auch aus Effluat aus entclippten Goldblattnieren sowie aus Überständen und Extrakten von interstitiellen Nierenmarkszellen gewonnen werden (Muirhead E.E., in "Textbook" o.a.O.).
*Bezüglich der physiologischen Eigenschaften:* Es senkt erhöhten Blutdruck, mit einer Verzögerung, innerhalb von wenigen Minuten. Dieser Effekt hält mehrere Stunden an (Muirhead E.E. in "Depressor Functions of the Kidney" Seminars in Nephrology, Vol. 3, No 1 (March), 14-29 (1983)). Nach Entnahme der Leber aus dem Kreislauf und nachfolgender Verabreichung von Medullipin hat das Medullipin keinerlei vasodepressorische Eigenschaften mehr (Muirhead E.E., in "Textbook" o.a.O.). Ebenso geht die Wirksamkeit nach vorheriger Gabe von Proadifen (SKF525A) verloren (Muirhead E.E., in "Textbook" o.a.O.).

Arbeiten, in denen blutdruckerhöhende Gemische oder Substanzen beschrieben wurden, waren:
1.) Die früheste Arbeit (Tigerstedt R., Bergmann P.G. in "Niere und Kreislauf", Skand. Arch. Physiol. 8, 223 (1898)), die zur Isolierung des selbst gefäßunwirksamen Renins führte (Haas E., Lamfrom H., Goldblatt H. in "Isolation and purification of hog renin" Arch. Biochem. Biophys. 42, 268, (1953)).
2.) Corticotensin: Dieses Peptid wurde als in der Schweinenierenrinde befindlich (Fasciolo, J.R., Risler N.R., Totel G: Corticotensins: Pressor Peptides from the kidney. In: Genest J und Koiw E (Herausgeber) Hypertension 72. Berlin. Springer, S172ff.) beschrieben, und erhöht den Blutdruck ebenfalls. Es ist bis heute nicht isoliert worden.
3.) Es ist immer wieder beschrieben worden (Abelous J.E., Bardier E: in "De l'action de l'extrait alcoolique de l'urine humaine normale sur la pression arterielle" CR. Soc. Biol. 64, 596, 868 (1908), Bain W. in "The pressor basis of urine" J. Exp. Physiol. 8, 229 (1914), Bohn H., Hahn F.Z. in "Untersuchungen zum Mechanismus des blassen Hochdrucks, blutdrucksteigernde Stoffe im Harn, insbesondere beim blassen und roten Hochdruck" Zeit. Klin. Med. 123, 558 (1933)), der Urin enthalte eine pressorisch wirkende Substanz. Diese Substanz wurde inzwischen als ein Protein von 25000 D Gewicht beschrieben, das bei chronischer Gabe hypertrophisch auf die Nebennierenrinde wirkt. Der Blutdruck steigt an, ebenso wie das Plasma-Aldosteron und die Na+/K+ Ratio im Urin (Sen S., Bravo E.L., Bumpus F.M. in "Isolation of a hypertension producing compound from human urine" Circ. Res. 50, (suppl 1) 5 (1977)).
4.) Renopressin: Es ist beschrieben worden, dass dieses Protein ebenfalls in der Schweinenierenrinde enthalten sei (Skeggs L.T., Kahn J.R., Levine M., Dorere F.E., Lentz K.E. in "Chronic one kidney hypertension in rabbits. III. Renopressin, a new hypertensive substance" Circ. Res. 40, 143 (1977)). Es handelt sich um Versuche, in denen gezeigt werden konnte, dass eine Zubereitung aus Reninfreier Schweinenierenrinde, den Blutdruck langsam erhöht, und diese Reaktion nicht durch Angiotensin II-Blocker antagonisierbar ist (Skeggs et al, o.a.O.)
5.) Nephrotensin: Aus dem venösen Effluat ischämischer Niere läßt sich ebenfalls eine blutdruckerhöhende Aktivität gewinnen (Grollmann A. und Krishnamurty V.S.R. in "Differentiation of nephrotensin from angiotensin I and II" Proc. Soc. Exp. Biol. Med. 143, 85 (1973).). Es wurde behauptet, dieses Protein sei an alpha-2-globulin gebundenes Angiotensin I (Schwikert J.R., Carey R.M., Liddle G.W., Islamnd D.P. in "Evidence that the renal pressor substance of Grollmann is related to angiotensin I" Circ. Res. 30, 31, 131 (1972)), die Aktivität bleibt jedoch nach der Gabe eines Antikörpers gegen Angiotensin I erhalten, und sie unterscheidet sich von der Aktivität von Angiotensin I und Angiotensin II bei der Ratte (Grollmann, o.a.O.).

Es war eine Aufgabe der vorliegenden Erfindung weitere vasoaktive Substanzen zur Verfügung zustellen. Überraschenderweise konnte durch ein besonderes Extraktionsverfahren mit sich anschließender Auftrennung eine bisher unbekannte blutdrucksenkende sowie eine blutdruckerhöhende Aktivität aus Säugetiernierenmark gewonnen werden, die mit bekannten blutdruckwirksamen Substanzen wie den Prostaglandinen E2, A2, Prostacyclin oder einem anderen Prostaglandin nicht identisch ist. Eine Identität mit dem bekannten Medullipin konnte ebenfalls ausgeschlossen werden.

### Beschreibung der Figuren

Figur 1 beschreibt die blutdrucksenkende Wirkung des Extraktes an der SH-Ratte bei einer Dosis von 10mg/kg Körpergewicht. Aufgetragen ist der arterielle Blutdruck (mmHg) über die Zeit in Minuten.
Figur 2 beschreibt die blutdrucksenkende Wirkung der Fraktion an der normotensiven Ratte bei einer Dosis von 10mg/kg Körpergewicht. Aufgetragen ist der arterielle Blutdruck (mmHg) über die Zeit in Minuten.
Figur 3 beschreibt die Dosisabhängigkeit des Blutdruckabfalles zwei Minuten nach der Gabe. Aufgetragen ist die Herabsetzung des systolischen und diastolischen Blutdrucks (in mmHg) bei verschiedenen Dosierungen (5, 10 und 20 mg/kg Körpergewicht).
Figur 4 beschreibt die Dosisabhängigkeit des Blutdruckabfalles eine Stunde nach der Gabe. Aufgetragen ist die Herabsetzung des systolischen und diastolischen Blutdrucks (in mmHg) bei verschiedenen Dosierungen (5, 10 und 20 mg/kg Körpergewicht).
Figur 5 beschreibt die blutdruckerhöhende Wirkung der Fraktion an der SH-Ratte bei einer Dosis von 10mg/kg Körpergewicht. Aufgetragen ist der arterielle Blutdruck (mmHg) über die Zeit in Minuten.
Figur 6 beschreibt die Wirkung des Lösungsmittels an der SH-Ratte. Aufgetragen ist der arterielle Blutdruck (mmHg) über die Zeit in Minuten.
Figur 7 beschreibt die Wirkung einer unwirksamen Vergleichsfraktion an der SH-Ratte. Aufgetragen ist der arterielle Blutdruck (mmHg) über die Zeit in Minuten.
Figuren 8a bis 8d zeigen die Massenspektren der als Vergleichsverbindungen eingesetzten Prostaglandine.
Figur 9 zeigt das Elutionsschema, die relativen Gewichte der einzelnen Fraktionen sowie die Dünnschichtchromatographiekarten bei der Gewinnung des blutdrucksenkenden Extrakts. TLC ist die Abkürzung für "Thin layer Chromatography". Darunter folgen die Fraktionsnummern als Zahlen. Darunter folgen die relativen Gewichte der einzelnen Fraktionen angegeben als Balkendiagramm. Darunter folgt die Angabe der verwendeten Lösemittelmengen für jede einzelne Fraktion.

### Beschreibung der Erfindung

### Blutdrucksenkender Extrakt

Die Erfindung betrifft somit zum einen ein Verfahren zur Isolierung einer blutdrucksenkenden Aktivität aus dem Nierenmark von Säugetieren durch
(i) Trocknung des Säugetiernierenmarks,
(ii) Extraktion des getrockneten Nierenmarks mit einem organischen Lösungsmittel mit einer Dielektrizitätskonstante zwischen 2 und 45,
(iii) Chromatographie des erhaltenen Extraktes über ein hydrophiles Sorbens mit einem Eluens-Gradienten von unpolar zu polar, und
(iv) Gewinnung der blutdrucksenkenden Aktivität als Extrakt nach Abtrennung des Elutionsmittels.

Im Zusammenhang mit der Erfindung ist die blutdrucksenkende Aktivität, Extrakt, Fraktion oder Substanz gleichbedeutend mit "Wirkstoff" zu verstehen.

Erfindungsgemäß kann das Säugetiernierenmark aus Schweinen, Kaninchen, Ratten, Schafen, Hunden, Rindern oder Primaten gewonnen werden.

Die Trocknung dient im wesentlichen der technologischen Verbesserung des späteren Extraktionsvorganges und ist dadurch gekennzeichnet, dass sie die schonende Entfernung des im Gewebe enthaltenen Wassers erlaubt. Dies geschieht vorzugsweise in Form einer Gefriertrocknung.

Das organische zur Extraktion des Nierenmarks eingesetzte Lösungsmittel weist vorzugsweise eine Dielektrizitätskonstante zwischen 45 und 2 auf. Dies entspricht Polaritätsindices zwischen 1,5 und 0 (nach: M.K. Gosh in "Methods on Drug Analysis", Springer Verlag, Heidelberg, 1992). Bevorzugt wird neben überkritischem Kohlendioxid auch ein C₁-C₃-Chlorkohlenwasserstoff, der ausgewählt ist aus der Gruppe bestehend aus Chloroform, Methylendichlorid, 1,1-Dichlorethan, 1,2-Dichlorethylen und 1,1,2-Trichlorethan. Besonders bevorzugt ist Chloroform.

Das Sorbens der Säulenchromatographie ist vorzugsweise eine Normalphase, ausgewählt aus der Gruppe bestehend aus den regulären oder irregulären Kieselgelen. Die Korngröße kann dabei 3 bis 500 µm, vorzugsweise 30 bis 200 µm und besonders bevorzugt 45 bis 100 µm betragen. Die Porengröße beträgt zwischen 4 bis 12 nm (40 und 120 Å), vorzugsweise zwischen 6 bis 12 nm (60-120 Å). Möglich ist aber gleichfalls der Einsatz anderer hydrophiler (=polarer) Sorbentien, underivatisierter oder derivatisierter Natur, die zur Normalphasenchromatographie geeignet sind.

Der Eluens-Gradient wird vorzugsweise hergestellt durch die Eluens-Abfolge C₅-C₁₀-Alkan, C₅-C₁₀-Alkan/C₂-C₈-Ether/Ethanol, C₂-C₈-Ether/Ethanol, C₂-C₈-Ether/Ethanol/Methanol, Ethanol/Methanol und Methanol, jeweils in steigend polaren Mischungsverhältnissen und Mischungen. Die organischen Lösungsmittel sind gleichfalls ersetzbar durch jeweilige Lösungsmittel ähnlicher Solvensstärke und Elutionskraft.

Die Mischungsverhältnisse (%V/%V) sind dabei vorzugsweise wie folgt: C₅-C₁₀-Alkan/C₂-C₈-Ether/Ethanol von 95/0/2 stufenweise nach 0/95/5, C₂-C₈-Ether/Ethanol von 95/5 stufenweise nach 20/80, C₂-C₈-Ether/Ethanol/Methanol von 20/80/0 stufenweise nach 0/50/50, Ethanol/Methanol von 50/50 stufenweise nach 0/100.

Das C₅-C₁₀-Alkan ist ein aliphatischer oder alicyclischer Kohlenwasserstoff und vorzugsweise ausgewählt aus der Gruppe bestehend aus, n-Pentan, n-Hexan, cylco-Hexan n-Heptan, n- oder iso-Oktan, n- oder iso-Nonan, n- oder iso-Dekan. Besonders bevorzugt ist n-Hexan.

Der C₂-C₈-Ether ist aliphatisch oder cycloaliphatisch und ist vorzugsweise ausgewählt aus der Gruppe bestehend aus Diethylether, Diisopropylether, Ethyl-isopropylether, tert-Butyl-methylether, Tetrahydrofuran und Dioxan. Besonders bevorzugt ist Diethylether.

Die bevorzugt einzusetzenden Alkohole sind ausgewählt aus der Gruppe der C₁-C₄-n- oder C₁-C₄-iso-Alkohole, besonders bevorzugt Ethanol, Methanol oder Gemische davon. Eine Substitution des Paares Ethanol/Methanol ist in analoger Kombination aus einem eher lipophilen und einem eher hydrophilen Alkohol möglich. Die blutdrucksenkende Aktivität findet sich in den polaren Fraktionen der Gradienten-Chromatographie, also in den Fraktionen, welche mit den genannten Alkoholen als Eluens erhalten werden.

Die hier beschriebene Säulenchromatographie lässt sich in analoger Weise unter Umkehrung der Polaritätsverhältnisse sowohl des Sorbens als auch des Gradienten gleichfalls mit Umkehrphasen als Sorbens betreiben. Besonders bevorzugt sind RP-18 und RP-8 Kieselgele mit einer Korngröße von 3 bis 500 µm, vorzugsweise 30 bis 200 µm und besonders bevorzugt 45 bis 100 µm. Die Porengröße beträgt zwischen 4 bis 12 nm (40 und 120 Å), vorzugsweise zwischen 6 bis 12 nm (60-120 Å). Möglich ist aber gleichfalls der Einsatz anderer hydrophiler (=polarer) Sorbentien. Ganz allgemein handelt es sich bei der Säulenchromatographie um eine schwerkraftgetriebene oder durch Unter- oder Überdruck forcierte Chromatographie in offenen oder geschlossenen Säulen.

Die Erfindung beschreibt ferner einen blutdrucksenkenden Extrakt erhältlich nach dem oben beschriebenen Verfahren, sowie eine pharmazeutische Zusammensetzung umfassend diesen blutdrucksenkenden Extrakt, oder im Sinne dieser Erfindung nicht vollständige Zubereitungen, insbesondere Zubereitungen die erhältlich sind unter Weglassen der Trocknung oder Weglassen der Chromatographie.

Der blutdrucksenkende Extrakt wird vorzugsweise in wässrige Phase eingebracht. Die verabreichungsfertige pharmazeutische Zubereitung kann neben dem blutdrucksenkenden Wirkstoff ferner Benetzungsmittel, Hilfssolventien, Lösungsvermittler und Stabilisatoren enthalten. Die Konzentrationen orientieren sich an der nachfolgend genannten, zu verabreichenden Dosis und dem Volumen oder Gewicht der Arzneiform. Die Verabreichung erfolgt systemisch oder peroral.

Der oben beschriebene blutdrucksenkende Extrakt und die oben beschriebene pharmazeutische Zusammensetzung kann zur Senkung des Blutdrucks bei Säugetieren, insbesondere beim Menschen verwendet werden. Dementsprechend kann der blutdrucksenkende Extrakt und die pharmazeutische Zusammensetzung umfassend diesen Extrakt zur Herstellung eines Medikaments zur Senkung des Blutdrucks bei Säugetieren verwendet werden.

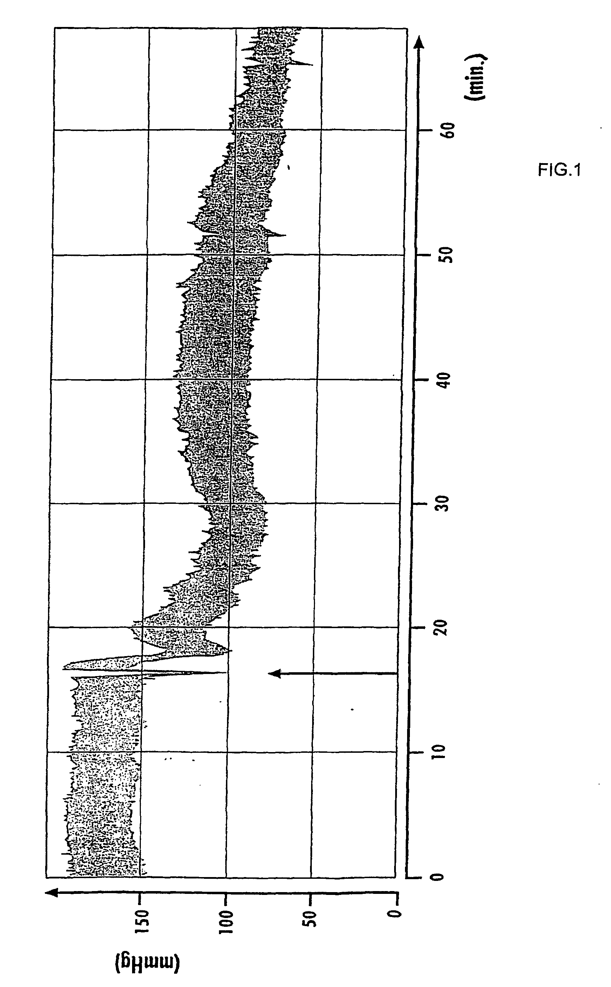

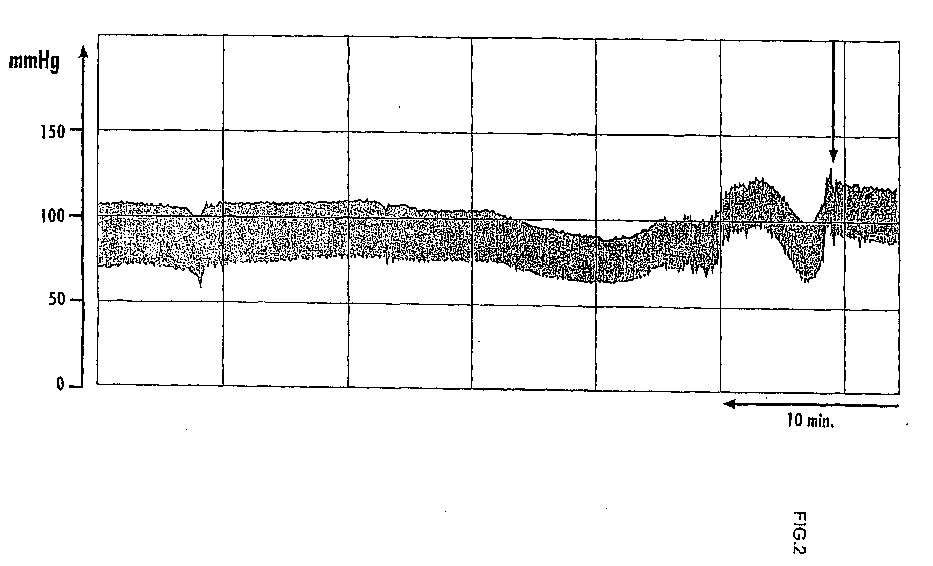

Die zu verabreichende Dosis beträgt 2 bis 50 mg/kg KG (KG = Körpergewicht), vorzugsweise 10 mg/kg KG, kann aber auch größer oder kleiner sein müssen. Die Wirkung einer Dosis von 10 mg/kg Körpergewicht zeigen die Figuren 1 und 2.

10 mg/kg Körpergewicht wurden einer spontan hypertensive Wistar-Ratte verabreicht. Den Zeitpunkt der Verabreichung zeigt der Pfeil an. Der Blutdruck sinkt ab, erreicht einen Tiefpunkt nach wenigen Minuten, steigt wieder etwas an und sinkt wieder. Der Blutdruck kann, nach einmaliger Applikation, für mehrere Stunden oder Tage niedrig bleiben. Diese Wirkung tritt analog an einem normotensiven Tier auf (Figur 1 zeigt den Blutdruckabfall bei einer SH-Ratte).

10 mg/kg Körpergewicht wurden einer normotensiven Ratte verabreicht. Den Zeitpunkt der Verabreichung zeigt der Pfeil an. Der Blutdruck sinkt ab, erreicht einen Tiefpunkt nach wenigen Minuten, steigt wieder etwas an und sinkt wieder. Es wird ein zweiter Tiefpunkt erreicht. Der Zeitraum bis zu diesem Tiefpunkt ist variabler als der Zeitraum bis zu dem ersten Tiefpunkt. Somit ist der Zeitraum des Abfalls des Blutdrucks bis zum folgenden Anstieg ebenfalls variabel. Hingewiesen werden soll aber auf die prinzipielle Ähnlichkeit der Blutdruckkurvenmorphologie in beiden Figuren. Der Verlauf des Blutdruckabfalls ist typisch. Der Blutdruck kann, nach einmaliger Applikation, für mehrere Stunden oder Tage niedrig gehalten werden. Die Abbildung in Figur 2 ist von rechts nach links zu lesen. Auf der Y-Achse ist der Blutdruck über der Zeit auf der X-Achse aufgetragen.

In einer Gruppe von sieben Tieren betrug der Blutdruckabfall nach dieser Dosis der Fraktion: 37,57 ± 22,14/28,57 ± 16,99 mmHg nach 2 Minuten, und 57,42 ± 29,07/45,.57 ± 22,41mmHg nach einer Stunde. Dieser Unterschied war mit einem p=0,0078 statistisch signifikant.

In einer Gruppe von sieben Tieren betrug der Blutdruckabfall nach dieser Dosis des Extraktes vor der Chromatographie: 20,71 ± 5,37/17,71 ± 6,72mmHg nach zwei Minuten, 29,57 ± 16,61/24,85 ± 14,69mmHg nach einer Stunde. Dieser Unterschied war mit einem p=0,0078 statistisch signifikant.

In einer Gruppe von sieben Tieren betrug der Blutdruckabfall nach der entsprechenden Dosis (1,5 ml) der Zubereitung ohne das Gemisch (Ethanol/Wasser): 0,71 ± 2,92/-0,14 ± 3,23 mmHg nach zwei Minuten, -1,14 ± 4,67/-1,71 ± 4,99 mmHg nach einer Stunde. Der Blutdruck blieb gleich.

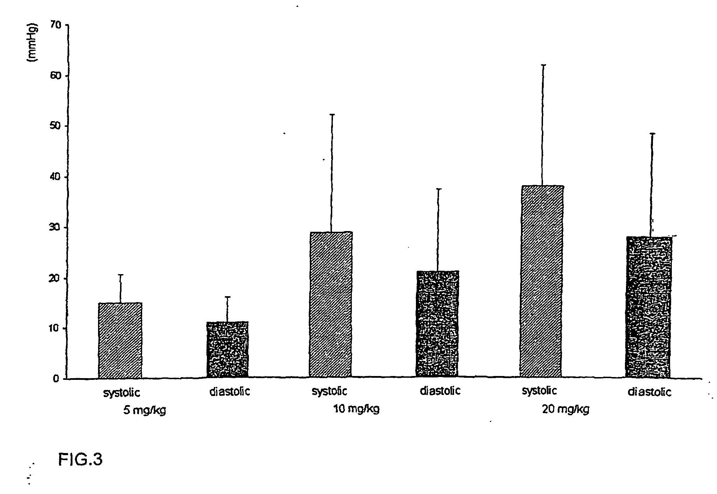

Figur 3 zwei zeigt die Dosisabhängigkeit des Blutdruckabfalls zwei Minuten nach der Gabe der Fraktion. Auf der y-Achse ist der Blutdruckabfall aufgetragen gegenüber den Dosierungen 5 (n=5), 10 (n=7) und 20 mg/kg KG (n=6) auf der x-Achse, aufgeteilt nach diastolischem und systolischem Blutdruck. Der Blutdruckabfall ist klar dosisabhängig.

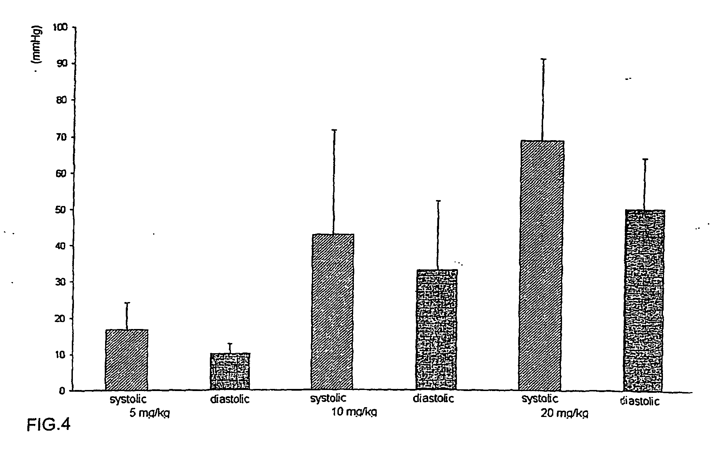

Figur 4 zeigt die Dosisabhängigkeit des Blutdruckabfalls eine Stunde nach der Gabe der Fraktion. Auf der y-Achse ist der Blutdruckabfall aufgetragen gegenüber den Dosierungen 5 (n=5), 10 (n=7) und 20 mg/kg KG (n=6) auf der x-Achse, aufgeteilt nach diastolischem und systolischem Blutdruck. Der Blutdruckabfall ist klar dosisabhängig.

Am gesamten Organismus bewirkt der Extrakt während der blutdrucksenkenden Wirkung keine Tachykardie. Es ist eine Bradykardie beobachtet worden.

Bei dem erfindungsgemäßen Extrakt handelt es sich um eine endogene Substanz eines Säugetieres. Es sind keine toxischen Wirkungen beobachtet worden.

Als weitere galenische Formen sind alle Formen von Injektabilia, orale Arzneiformen einschließlich solcher mit geänderter Wirkstoff-Freisetzung und Arzneiformen auf der Basis von Nanopartikeln, denkbar.

Die photonen-korrelations-spektroskopisch bestimmte Turbidität des injizierbaren Kolloids des Extraktes, das wie unten beschrieben zubereitet worden war, wurde durch Tröpfchen mit einer Größe von 143 ± 47 nm () verursacht. Das Kolloid, das aus der Fraktion 47 bis 73 zubereitet worden war, hatte eine mittlere Tröpfchengröße von 188 ± 12 nm (). Die Partikel hatten somit eine Tröpfchengröße, die weit unter der Toleranzgrenze der Lungengängigkeit lag, und war somit für den Versuch galenisch einwandfrei.

Gemäß der vorliegenden Erfindung wird gezeigt, dass ein, wie oben beschrieben, chromatographisch aufgearbeiteter, chloroformischer Nierenmarksextrakt eines Säugetieres, insbesondere des Schweins, bei einer Nachweisgrenze von besser als 3 ppm frei von Prostaglandinen ist, gleichzeitig aber antihypertensive Eigenschaften besitzt. Umgerechnet auf die Tiere, kann ein Tier von einem Prostaglandin nicht mehr als ~0.6⁻¹⁰ M erhalten haben können. Diese Menge ist in Bezug auf den Blutdruck irrelevant, und erklärt den hier gezeigten Effekt nicht. Dadurch sind Zweifel, ob es sich bei allen beobachteten blutdrucksenkenden Aktivitäten nicht doch um Aktivitäten handeln könnte, die durch Prostaglandine verursacht werden, ausgeräumt. Erfindungsgemäß tritt diese Aktivität beinahe akut ein, hat einen Wirkgipfel bei etwa 10 Minuten nach der Injektion, und hält insgesamt über mehr als eine Stunde an. Für die erfindungsgemäße Aktivität gibt es keinen typischen Wirkverlauf, der einer bekannten Substanz zuzuordnen sein könnte.

Der erfindungsgemäße blutdrucksenkende Wirkstoff kann somit zur dosisabhängigen Senkung des Blutdrucks bei Säugetieren oder zur Erzielung von mit dieser Wirkung zusammenhängenden Effekten, wie insbesondere der Entlastung des Herzens und Vorbeugung oder Behandlung anderer Krankheiten des Kreislaufes, der Gefäße einschließlich des Herzens eingesetzt werden, gleichgültig ob der Ausgangsblutdruck ein hypertoner Blutdruck ist oder ein normotoner Blutdruck. Eine kontinuierliche Infusion des erfindungsgemäßen Wirkstoffs ist nicht notwendig.

Keine der blutdrucksenkenden Gemische oder Substanzen des Standes der Technik kommt in Frage, die erfindungsgemäß beobachtete Aktivität zu erklären.

Ebenfalls nicht in Frage für die blutdrucksenkende Aktivität kommen prostanoide Substanzen, die in außerordentlich großer Menge im Nierenmark enthalten sind (Daniels et al., o.a.O.). Diese aus dem Nierenmark isolierten Prostaglandine, Prostaglandin E2 und A2, gehören jedoch zu den stärksten vasodilatorischen Agenzien, die bekannt sind. Es konnte jedoch ausgeschlossen werden, dass sie für die hier beschriebene Aktivität in Frage kommen, indem gezeigt wurde, dass sie nicht in ausreichender Menge in dem erfindungsgemäßen Extrakt vorhanden sind.

Um Prostaglandine aus dem Nierenmark zu isolieren (Lee J.B., Crowshaw K., Takman B.H., Attrep K.A. in "The identification of prostaglandins E2, F2α and A2 from Rabbit Kidney Medulla" Biochem. J. 105; 1967: 1251-1260) wurde folgende Methode der Lipidextraktion angewendet, wie Lee ausführt (Lee J.B. in "Prostaglandins, neutral lipids, renal interstitial cells and hypertension" Genest. J., Koiw E., Kuchel O. (Herausgeber) in "Hypertension: Physiopathology and treatment" McGraw-Hill, New York, 1977, S. 373 - 390), um neutrale Lipide auszuschließen und die sauren Prostaglandine zu erhalten und gleichzeitig von den Neutrallipiden abzutrennen. Es wurde zunächst angesäuertes wässriges Homogenisat des Nierenmarkes mit Methylenchlorid extrahiert. Man erwartete, dass sowohl die sauren als auch die neutralen Lipide enthalten sein würden. Das Methylenchlorid wurde dann gegen einen Phosphatpuffer ausgeschüttelt unter der Vorstellung, dass nur die sauren, nicht aber die neutralen Lipide in den Puffer übergehen würden. Der Phosphatpuffer wurde sodann angesäuert und wiederum mit Methylenchlorid extrahiert; diese Lösung enthielt dann die Aktivität, die später zur Auffindung der genannten Prostaglandine in der Niere führte (Lee J.B., Crowshaw et al, o.a.O.).

Würde der hier beschriebene Extrakt prostanoide Substanzen enthalten, wäre eine diesen Substanzen zuordenbare Aktivität in der Fraktion 47 bis 73, analog zu der Arbeit von Lee, zu erwarten gewesen. Dies allerdings konnte erfindungsgemäß nicht beobachtet werden. Nachdem gezeigt worden ist, dass das Nierenmark eine erhebliche Menge besonders vasodilatatorischer Prostaglandine enthält, erfindungsgemäß aber keine dieser Substanzen in ausreichender Menge nachgewiesen wurde und keine diesen Substanzen zuordenbare blutdruckbezogene Aktivität gefunden wurde, ist es ausgeschlossen, dass in der erfindungsgemäß beschriebenen Fraktion die erfindungsgemäß blutdrucksenkende Aktivität auf die Anwesenheit prostanoider Substanzen zurückzuführen ist.
- Das blutdrucksenkende Extrakt ist nicht auf vasodilatatorische und/oder blutdrucksenkende prostanoide Substanzen zurückzuführen. Die Anwesenheit dieser Substanzen ist, wie gezeigt, ausgeschlossen worden.
- Die Aktivität des erfindungsgemäßen blutdrucksenkenden Extrakts ist nicht auf die Anwesenheit eines Gemisches namens APRL zurückzuführen, da APRL in dem erfindungsgemäßen Extrakt, nicht vorhanden ist (Prewitt R.L., Leach B.E., Byers L.W., Brooks B., Lands W.E.M., Muirhead E. in "Antihypertensive polar renomedullary lipid, a semisynthetic vasodilator" Hypertension 1, 1979: 299-308). Der Grund dafür ist, dass APRL ein halbsynthetisches Gemisch aus Lipiden darstellt.
- Die Aktivität des erfindungsgemäßen blutdrucksenkenden Extrakts ist nicht auf die Anwesenheit von Medullipin zurückzuführen, da
   - Medullipin als AN(neutral)RL beschrieben worden ist (Muirhead, in "Textbook" o.a.O.), die erfindungsgemäße Aktivität aber als polar bis hochpolar zu bezeichnen ist.
   - Medullipin mit Chloroform von einer Kieselgelsäule eluiert wird, und in den Anfangsfraktionen zu finden ist (Muirhead E.E., Byers L.W., Desiderio E.M., Pitcock J.A., Brooks B., Brown P.S., Brosius W.L. in "Derivation of antihypertensive neutral renomedullary lipid from renal venous effluent" J. Lab. Clin. Med. 99; 1982: 64-74).
   - Medullipin mit der Methode von Bligh und Dyer (Bligh E.G., und Dyer W.J. in "A Rapid Method of total lipid extraction and purification", Canadian Journal of Biochemistry and Physiology. 37; (8) 1959) extrahiert wird, die erfindungsgemäße blutdrucksenkende Aktivität aber mit Chloroform extrahiert wird.
   - Ein Extrakt, welcher Medullipin enthält, entfaltet entweder keine gefäßwirksame oder eine vasodilatatorische Aktivität (Muirhead E.E., in "Textbook" (o.a.O) und Muirhead E.E. in "Depressor Functions of the Kidney" Seminars in Nephrology, Vol 3, No 1 (March), 14-29 (1983)). Die vasodilatatorische Wirkung, auf welche die blutdrucksenkende Wirkung von Medullipin zurückgeführt wird, ist jedoch nie gezeigt worden. Der erfindungsgemäße blutdrucksenkende Extrakt zeichnet sich jedoch durch eine starke dilatorische Wirkung an der Aorta der Ratte und Koronarien des Schweins aus.
   - Der erfindungsgemäße blutdrucksenkende Extrakt kann den Agenzien zugeordnet werden, welche die stärkste bekannte blutdrucksenkende Aktivität beim Säugetier besitzen. Den Blutdruck beinahe unmittelbar, stark, langanhaltend zu senken, ist eine einzigartige Eigenschaft, die der erfindungsgemäße blutdrucksenkende Extrakt auch von allen anderen bekannten Wirkstoffen oder Extrakten unterscheidet.
   - Der erfindungsgemäße blutdrucksenkende Extrakt ist direkt dosisabhängig blutdrucksenkend wirksam. Dies unterscheidet es von Medullipin oder anderen Nierenmarksextrakten, für die eine solche Eigenschaft nicht bekannt ist.
   - Der erfindungsgemäße blutdrucksenkende Extrakt senkt den Blutdruck nicht nur an hypertensiven Individuen, sondern auch, ähnlich stark, an normotensiven Individuen, lediglich von der Dosierung abhängig. Eine solche Eigenschaft ist sonst nur dem Nitroprussid-Natrium zu eigen; allerdings muss dieses kontinuierlich infundiert werden, d.h. der erfindungsgemäße Extrakt hat eine lange Wirkung, die ihn vom einzigen vergleichbaren Wirkstoff, Nitroprussid-Natrium, unterscheidet und ihm gegenüber einen entscheidenden Vorteil bietet. Auch dies wird dem Medullipin nicht zugeschrieben.
   - Die im Zusammenhang mit dem blutdrucksenkenden Extrakt gefundene Blutdruckkurvenmorpholgie, d. h. der Zeitverlauf des Blutdruckabfalles, ist einzigartig. Er unterscheidet sich von allen bekannten Wirkstoffen oder Extrakten:

   - Von den Prostaglandinen, da diese einen sofortigen (in den ersten Sekunden nach der Gabe) Blutdruckabfall bewirken, dessen Tiefpunkt sehr schnell eintritt, nach Sekunden bis mehreren Sekunden. Wie in den Figuren gezeigt, erreicht die erfindungsgemäße Aktivität erst nach Minuten einen Höhepunkt (=den ersten Tief-punkt des Blutdruckes), steigt wieder an, und fällt zu einem zweiten Tiefpunkt ab. Das bewirken prostanoide Substanzen, oder irgendwelche anderen bekannten Substanzen nicht. Prostaglandine verlieren ihre Wirkung nach wenigen Minuten (Fitzpatrick T.H., Alter I., Corey E., Ramwell P., Rose J.C., Kot P. in Circulation Research 42; 1978: 192-194), die erfindungsgemäße blutdrucksenkende Aktivität nicht. Darüberhinaus ist die Dosis, die erfindungsgemäß verabreicht wurde, ohne sie mittels GCMS nachgewiesen zu haben, weil sie unter der Nachweisgrenze lag, etwa drei Zehnerpotenzen niedriger als die, die benötigt wird, um mit Prostaglandinen einen Blutdruckeffekt zu erzeugen (Fitzpatrick T.H., o.a.O.).
   - Vom Medullipin, weil ein Medullipinextrakt nicht sofort wirksam ist (mit einer Zeitverzögerung von Minuten), und ein Medullipinextrakt nur einen Tiefpunkt erreicht (Muirhead E.E., in "Textbook" 0.a.O. und Muirhead E.E. in "Depressor Functions of the Kidney" Seminars in Nephrology, Vol 3, No 1 (March), 14-29 (1983)).
   - Der erfindungsgemäße blutdrucksenkende Extrakt hat keine Tachykardie ausgelöst. Diese Eigenschaft ist keiner bekannten körpereigenen oder natürlichen Substanz oder keinem solchen Gemisch zu eigen, die blutdrucksenkend ist. Das blutdrucksenkende Extrakt soll nachfolgend den Namen "Angiolysin" tragen.

### Blutdruckerhöhender Extrakt

Die Erfindung betrifft zum anderen ein Verfahren zur Isolierung einer blutdruckerhöhenden Aktivität aus dem Nierenmark von Säugetieren durch
(i) Trocknung des Säugetiernierenmarks,
(ii) Extraktion des getrockneten Nierenmarks mit einem organischen Lösungsmittel mit einer Dielektrizitätskonstante zwischen 2 und 45,
(iii) Chromatographie des erhaltenen Extraktes über ein hydrophiles Sorbens mit einem Eluens-Gradienten von unpolar zu polar,
(iv) Gewinnung der blutdrucksenkenden Aktivität als Extrakt nach Abtrennung des Elutionsmittels und
(v) Gewinnung der blutdruckerhöhenden Aktivität durch Gelchromatographie des Rückstandes mit einem organischen Lösungsmittel.

Im Zusammenhang mit der Erfindung ist die blutdruckerhöhende Aktivität, Extrakt oder Substanz gleichbedeutend mit "Wirkstoff" zu verstehen.

Zu den Quellen des Nierenmarks, dem zu dessen Extraktion verwendeten organischen Lösungsmittels, der Durchführung der Gradienten-Chromatographie und zur Elution des Rückstandes vor der sich anschließenden Gelchromatographie wird auf die obigen Ausführungen, die im Zusammenhang mit dem blutdrucksenkenden Extrakt gemacht sind, Bezug genommen. Der der Gelchromatographie zu unterwerfende Rückstand wird aus den oben beschriebenen polaren Fraktionen (Eluat-Fraktionen) der Gradienten-Chromatographie erhalten.

Zur Gelchromatographie können quellbare Polysaccharide, insbesondere vom Agarose- und Sepharose-Typ als Träger verwendet werden. Bevorzugt ist lipophilisierte und/oder quervernetzte Sepharose vom Sephadex®-Typ, wie Sephadex® LH-20. Als Eluens wird organisches Lösungsmittel, vorzugsweise mit einer Dielektrizitätskonstante zwischen 2 und 45, entweder in Reinform oder als Gemisch eingesetzt. Als Eluens kommen Lösungsmittel in Frage die ausgewählt sind aus der Gruppe bestehend aus C₁-C₄-Alkoholen, C₂-C₈-Ethern, C₅-C₁₀ aliphatischen Alkanen, C₆-C₁₂ aromatischen Kohlenwasserstoffen, C₃-C₉ Ketonen und deren Gemischen. Vorzugsweise eignen sich C₁-C₄-n- oder C₁-C₄-iso-Alkohole, insbesondere Ethanol oder Methanol sowie C₁-C₃-Chlorkohlenwasserstoffe. Besonders bevorzugt sind Mischungen von C₁-C₄-n- oder C₁-C₄-iso-Alkoholen, insbesondere Ethanol oder Methanol mit einem C₁-C₃-Chlorkohlenwasserstoff im Mischungsverhältnis 0-100/0-100, vorzugsweise 30-70/30-70. Der C₁-C₃-Chlorkohlenwasserstoff ist aus der oben beschriebenen Gruppe ausgewählt. Besonders bevorzugt ist ein Gemisch aus Methanol und Dichlormethan im Verhältnis 1:1.

Die Erfindung betrifft ferner einen blutdruckerhöhenden Extrakt erhältlich nach dem oben beschriebenen Verfahren, sowie eine pharmazeutische Zusammensetzung umfassend dieses blutdruckerhöhenden Extrakts.

Der blutdruckerhöhende Extrakt wird vorzugsweise in wässrige Phase eingebracht. Die verabreichungsfertige pharmazeutische Zusammensetzung kann neben dem blutdruckerhöhenden Wirkstoff ferner Benetzungsmittel, Hilfssolventien, Lösungsvermittler und Stabilisatoren enthalten. Die Konzentrationen orientieren sich an der untengenannten, zu verabreichenden Dosis und dem Volumen oder Gewicht der Arzneiform. Die Verabreichung erfolgt systemisch oder peroral.

Der oben beschriebene blutdruckerhöhende Extrakt bzw. die oben beschriebene pharmazeutische Zusammensetzung kann zur Erhöhung des Blutdrucks bei Säugetieren, insbesondere bei Menschen verwendet werden und dem entsprechend wird der blutdruckerhöhende Extrakt bzw. die pharmazeutische Zusammensetzung zur Herstellung eines Medikaments zur Erhöhung des Blutdrucks bei Säugetieren verwendet. Die zu verabreichende Dosis beträgt 2-100 mg/kg KG, vorzugsweise 10 mg/kg KG, kann aber auch größer oder kleiner sein, je nach Reaktion des Patienten.

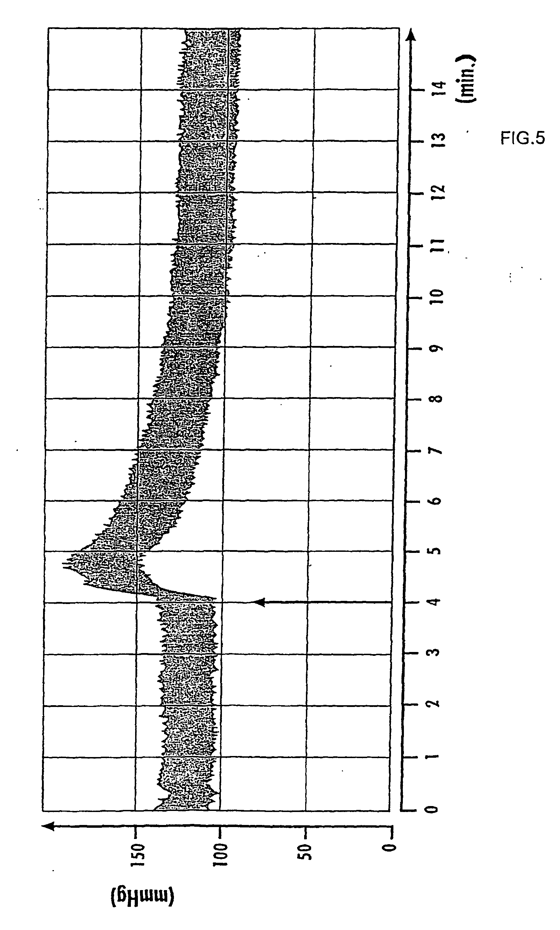

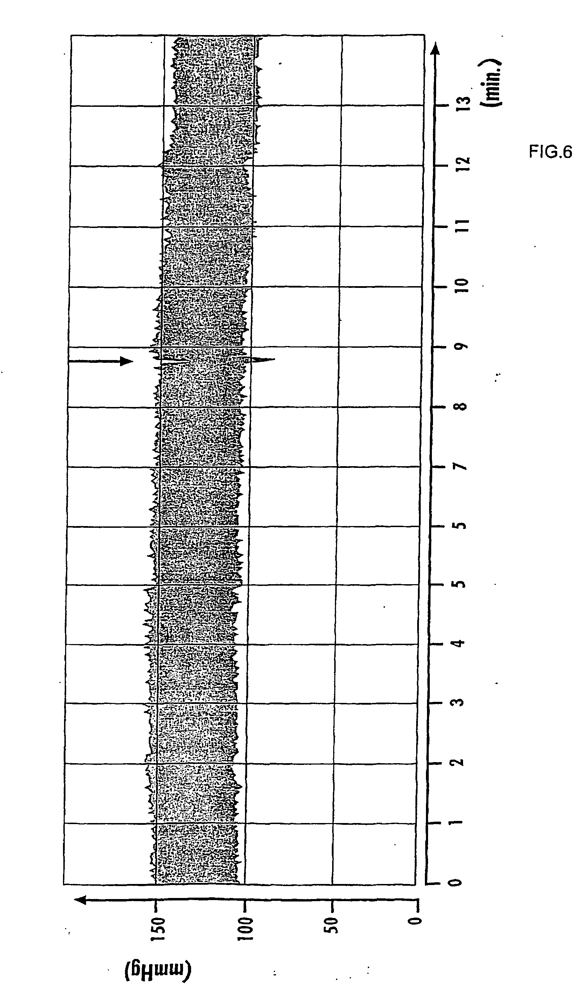

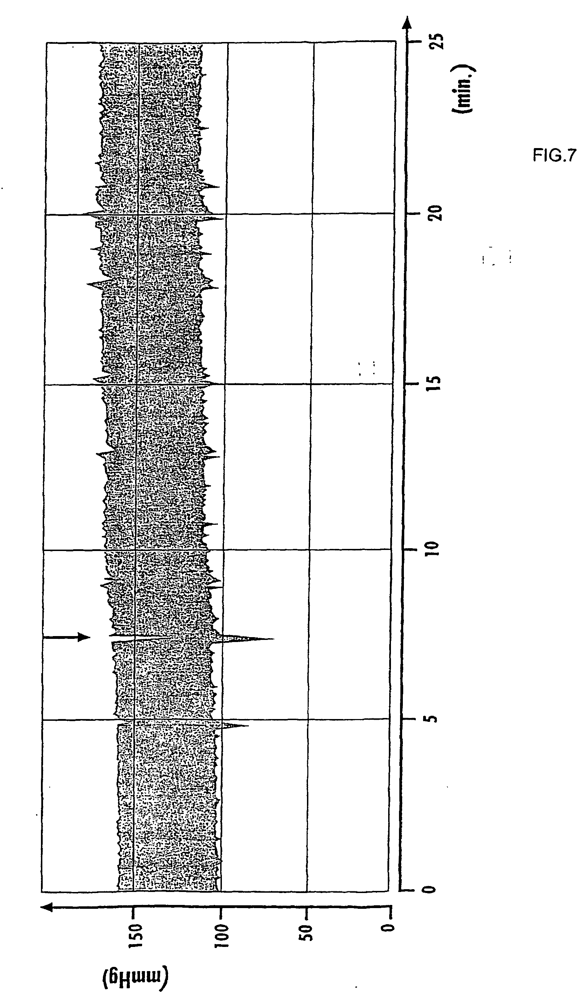

Die Wirkung einer Dosis von 10 mg/kg Körpergewicht zeigt Figur 5. Es tritt ein sofortiger Blutdruckanstieg auf, der für wenige Minuten anhält. Die Wirkung des entsprechenden Lösemittels zeigt Figur 6. Die Wirkung einer Vergleichsfraktion, die unwirksam ist, zeigt die Figur 7.

Die gezeigten Wirkungen, blutdruckerhöhend wie blutdrucksenkend, sind also nicht darauf zurückzuführen, dass ein Volumeneffekt des Lösemittels vorliegt, wie hier gezeigt. Die gezeigten Wirkungen, blutdrucksenkend wie blutdruckerhöhend, sind nicht auf Eigenheiten der Zubereitung zurückzuführen, wie hier gezeigt.

Die Beschreibung der Gruppen, die gebildet worden sind, ist wie folgt:
1.) Fraktion 1 bis 276: Diese Fraktion zeigt eine ausgeprägte blutdruckerhöhenden Aktivität, die ihr Maximum bei 55 ± 27 Sekunde nach Beendigung der Injektion erreicht und 201 ± 59 Sekunden andauert. Ein Beispiel für diesen kurzanhaltenden Blutdruckanstieg zeigt Figur 1. Wie daraus ersichtlich steigt der Blutdruck nahezu sofort nach der Injektion kontinuierlich an, erreicht sein Maximum und fällt etwas langsamer wieder ab auf den ursprünglichen Level. der Blutdruckanstieg betrug 30,1 ± 7,1 mmHg systolisch und 34,7 ± 6 mmHg diastolisch (p=0,0003).
2.) Fraktion 1 bis 48 (Vergleichsfraktion): Nach Injektion dieser Fraktion ergab sich keine nennenswerte Veränderung des Blutdrucks. Ein Beispiel zeigt Figur 2, die ebenso belegt, dass keine sichtbare Veränderung stattfindet. Zu demjenigen Zeitpunkt, der der nach Injektion von Fraktion 1 bis 276 beobachteten Blutdruckspitze, also 55 Sekunden nach Injektion, entspricht, wurde bei der Vergleichsfraktion lediglich ein geringer und statistisch unsignifikanter Abfall im systolischen Druck um 1,1 ± 3 mmHg, begleitet von einem geringen und statistisch unsignifikanter Abfall im diastolischen Druck um 0,3 ± 5,1 mmHg gemessen.
3.) Lösungsmittel-Kontrolle: Nach Injektion einer Mischung aus Ethanol und Wasser, so, wie sie für die Zubereitung der Injektabilia der Proben nach 1. und 2. verwendet wurde, ergab sich ebenfalls kein nennenswerter Blutdruckeffekt. Ein Beispiel zeigt Figur 2. Zu demjenigen Zeitpunkt, der der nach Injektion von Fraktion 1 bis 276 beobachteten Blutdruckspitze, also 55 Sekunden nach Injektion, entspricht, wurde bei der Lösungsmittel-Kontrolle lediglich ein geringer und statistisch unsignifikanter Abfall im systolischen Druck um 0,1 ± 3 mmHg, begleitet von einem geringen und statistisch unsignifikanter Abfall im diastolischen Druck um 0,9 ± 2,2 mmHg gemessen.

Weitere galenische Formen sind alle Formen von Injektabilia, orale Arzneiformen einschließlich solcher mit geänderter Wirkstoff-Freisetzung und Arzneiformen auf der Basis von Nanopartikeln.

Die erfindungsgemäße blutdruckerhöhende Aktivität repräsentiert ein im Nierenmark der obengenannten Säugetiere, insbesondere des Schweins enthaltenes Lipid, das den Blutdruck für wenige Minuten zu erhöhen vermag. Ein solches blutdruckerhöhendes Lipid ist, bis auf den geaschromatographischen Nachweis von Thromboxan A2 (Goswami S., Mai J., Bruckner G., Kuinsella J.E. in "Extraction and Purification of prostaglandins and thromoxane from biological samples for gas chromatographc analysis" Prostaglandins 22; 1981, 693-702), bis heute als in der Niere vorkommend nicht beschrieben worden.

In allen anderen Fällen handelt es sich um Proteine, die teilweise sogar von recht hohem Molekulargewicht sind. Erfindungsgemäß wurde das Nierenmark jedoch mit reinem Chloroform extrahiert; dies entspricht nicht der Methode der Lipidextraktion und Aufreinigung von Bligh und Dyer (o.a.O), bei der aus einem monophasischen Gemisch von Gewebswasser, Methanol und Chloroform, in dem das Gewebe homogenisiert wird, durch Zugabe von Wasser und Chloroform ein biphasisches Gemisch hergestellt wird, wobei die chloroformische Schicht die Lipide und die Methanol/Wasser Schicht alle anderen Substanzen enthielt, nach dr Muirhead arbeitete.

Als für die erfindungsgemäß beschriebene blutdruckerhöhende Aktivität verantwortlich in Frage könnte das Thromboxan A2 als endogenes, von Arachidonsäure abgeleitetes Peroxid in Frage kommen. Die erfindungsgemäß isolierte Aktivität ist mit seiner blutdruckerhöhenden Eigenschaft in Ausmaß und Zeitdauer, trotz einiger Abweichungen, besonders dem schnelleren Blutdruckanstieg nach Verabreichung von Thromboxan A2 am ehesten vergleichbar ist (Svensson, J. in "Biosynthesis and biological properteies of prostaglandin endoperoxids and thromboxane A2", Acta Biol. Med. Germ. 37, 1978: 731-740). Aufgrund seiner geringen Stabilität mit einer Halbwertszeit von nur etwa 30 Sekunden, scheidet es als Kandidat jedoch sicher aus. Unter den erfindungsgemäßen Bedingungen zur Isolierung und Verarbeitung wäre Thromboxan A2 sicher zerstört worden (Svensson et al., o.a.O.).
- Der erfindungsgemäße blutdrucksteigernde Extrakt ist nicht von den Katecholaminen abgeleitet und steigert den Blutdruck sofort und kurz, aber stark. Die macht ihn bei der Anwendung hervorragend steuerbar.
- Der erfindungsgemäße blutdruckerhöhende Extrakt bewirkt eine Erhöhung des Blutdrucks auch bei normalem oder niedrigem Blutdruck.
- Die Morphologie der Blutdruckkurve, d.h. der Zeitverlauf des Blutdruckanstieges, ist einzigartig und bis jetzt nicht beschrieben worden. Das blutdruckerhöhende Extrakt soll nachfolgend den Namen "Medullopressin" tragen.

Die nachfolgenden Beispiele beschreiben die Erfindung ohne sie jedoch zu beschränken.

### Beispiele

### 1. Blutdrucksenkende Aktivität (Angiolysin)

### Material und Methoden

### (i) Gewinnung des Nierenmarkes

Das Nierenmark wurde aus Schweinenieren gewonnen. Etwa 15 Minuten nach der Schlachtung der Tiere, unmittelbar nach der Fleischbeschau, wurden die Nieren vom Lunge-Leber-Nierenpaket am Fließband mit einem Messer abgetrennt und sofort in gestoßenes Eis gelegt. Etwa 45 Minuten später wurden sie in flüssigem Stickstoff schockgefroren und bei -40°C bis zur Dissektion aufbewahrt.

Zur Dissektion wurden die Nieren bei Raumtemperatur bis zum Erreichen einer Rindentemperatur von -5 bis -2 °C erwärmt. In diesem Zustand war es möglich, sie ohne Auftauen auf einem herkömmlichen elektrischen Allesschneider der Länge nach in etwa 2-3 mm dicke Scheiben zu schneiden.

Aus den Nierenscheiben wurde möglichst schnell sehr sorgfältig das Nierenmark mit Skalpellen von Nierenbecken und von Nierenrinde getrennt und sofort wieder in flüssigen Stickstoff gelegt. Anschließend wurde es wiederum bei -40 °C aufbewahrt. Es wurden auf diese Weise 23,42 kg Nierenmark aus etwa 2500 Nieren (800 kg) gewonnen.

Das Nierenmark wurde anschließend pulverisiert. Dazu wurde es in gefrorenem Zustand in kleinen Portionen zu jeweils 80 bis 100 g in den stickstoffgekühlten Stahlbecher eines Küchenmixers gegeben. Auf das Nierenmark wurde in den Stahlbecher ebenfalls flüssiger Stickstoff gegeben. Sobald dieser sich verflüchtigt hatte, wurde das Nierenmark pulverisiert. Mit dem gesamten Material wurde portionsweise so verfahren. Das pulverisierte Nierenmark wurde wiederum bei -40°C aufbewahrt.

Anschließend wurde das Pulver auf herkömmliche Weise gefriergetrocknet. Die Trockenmasse des gefriergetrockneten Materials betrug 3,62 kg.

### (ii) Extraktion

Hierzu wurden 1585,5 g des Lyophilisates in ein 20 Liter Becherglas überführt, mit Chloroform übergossen und mit einem Mischstab für etwa 10 Minuten auf höchster Stufe vermischt. Anschließend erfolgte die Entfernung der Schwebstoffe durch Filtration mit einem herkömmlichen Papierfilter. Das abgefilterte Material wurde nun auf dieselbe Weise weitere neun Mal mit insgesamt etwa 75 Liter Chloroform extrahiert, bis das Filtrat, das bei den ersten Extraktionsstufen von dunkelgelblicher Farbe war, farblos geworden war. Die erhaltene Lösung wurde sodann bei Raumtemperatur in vacuo zur Trockne gebracht. Es blieben 204,9 g eines sehr dickflüssigen, dunkelgelblichen Öls zurück. Zu Testzwecken wurde davon eine Probe von 2 g Gewicht entnommen. Das Material wurde sodann mit etwas Chloroform auf 259,9 g irreguläres Kieselgel der Firma ICN mit einer Korngröße von 32 bis 64 µm und einer Porengröße von 600 nm (60 Å) aufgezogen und in einem Exsiccator wieder von Chloroform befreit. Es wurden 464 g eines trockenen Pulvers erhalten, das bis zur Weiterverarbeitung bei -80 °C aufbewahrt wurde.

### (iii) Gradienten-Chromatographie

Um die Trennung nicht auf mehrere Schritte aufteilen zu müssen, die dann erforderlichen Säulendimensionen aber eine schwerkraftgetriebene Elution nicht mehr zulassen, wurde zur Elution eine Vakuumchromatographiesäule entworfen und konstruiert. In dieser Säule kann mit Hilfe eines Vakuums so eluiert werden, dass das Verhältnis Sorbens zu Probe zugunsten der eingesetzten Sorbensmenge vergrößert werden kann. Die verwendete Säule hatte bei eine Höhe von 400 mm einen Innendurchmesser von 16 mm, und wurde mit 2204 g Kieselgel der Firma ICN mit einer Korngröße von 32 bis 64 µm und einer Porengröße von 600 nm (60 Å) gefüllt. Auf den Extrakt wurden ein Papierfilter und 200 g Seesand gelegt.

Eluiert wurde in 73 Schritten über einen Gradienten, beginnend mit Hexan über Hexan-Diethylether, Hexan-Diethylether-Ethanol, Diethylether-Ethanol, Diethylether-Ethanol-Methanol, Ethanol-Methanol, reinem Methanol, zu Chloroform.

### (iv) Elution der Säule

Die Fraktion 73 bestand aus reinem Chloroform, da mit Chloroform extrahiert worden war. Begonnen wurde mit reinem Hexan. Es wurden 73 Fraktionen gewonnen. Die genauen Volumina der 73 Fraktionen zeigt folgende Tabelle:

**Tabelle**

| | Hexan (ml) | Diethylether (ml) | Ethanol (ml) | Methanol (ml) | Chloroform (ml) |
|---|---|---|---|---|---|
| Fraktion 1 | 1500 | 0 | 0 | 0 | 0 |
| Fraktion 2 | 980 | 20 | 0 | 0 | 0 |
| Fraktion 3 | 970 | 30 | 0 | 0 | 0 |
| Fraktion 4 | 960 | 40 | 0 | 0 | 0 |
| Fraktion 5 | 950 | 50 | 0 | 0 | 0 |
| Fraktion 6 | 940 | 60 | 0 | 0 | 0 |
| Fraktion 7 | 1395 | 105 | 0 | 0 | 0 |
| Fraktion 8 | 1380 | 120 | 0 | 0 | 0 |
| Fraktion 9 | 1365 | 135 | 0 | 0 | 0 |
| Fraktion 10 | 1350 | 150 | 0 | 0 | 0 |
| Fraktion 11 | 890 | 100 | 10 | 0 | 0 |
| Fraktion 12 | 880 | 110 | 10 | 0 | 0 |
| Fraktion 13 | 870 | 120 | 10 | 0 | 0 |
| Fraktion 14 | 1290 | 195 | 15 | 0 | 0 |
| Fraktion 15 | 1275 | 210 | 15 | 0 | 0 |
| Fraktion 16 | 840 | 150 | 10 | 0 | 0 |
| Fraktion 17 | 830 | 160 | 10 | 0 | 0 |
| Fraktion 18 | 820 | 170 | 10 | 0 | 0 |
| Fraktion 19 | 810 | 180 | 10 | 0 | 0 |
| Fraktion 20 | 800 | 190 | 10 | 0 | 0 |
| Fraktion 21 | 790 | 200 | 10 | 0 | 0 |
| Fraktion 22 | 780 | 210 | 10 | 0 | 0 |
| Fraktion 23 | 760 | 230 | 10 | 0 | 0 |
| Fraktion 24 | 730 | 260 | 10 | 0 | 0 |
| Fraktion 25 | 700 | 280 | 20 | 0 | 0 |
| Fraktion 26 | 680 | 300 | 20 | 0 | 0 |
| Fraktion 27 | 660 | 320 | 20 | 0 | 0 |
| Fraktion 28 | 630 | 340 | 30 | 0 | 0 |
| Fraktion 29 | 620 | 350 | 30 | 0 | 0 |
| Fraktion 30 | 600 | 370 | 30 | 0 | 0 |
| Fraktion 31 | 580 | 390 | 30 | 0 | 0 |
| Fraktion 32 | 560 | 410 | 30 | 0 | 0 |
| Fraktion 33 | 540 | 420 | 40 | 0 | 0 |
| Fraktion 34 | 520 | 440 | 40 | 0 | 0 |
| Fraktion 35 | 750 | 690 | 60 | 0 | 0 |
| Fraktion 36 | 480 | 480 | 40 | 0 | 0 |
| Fraktion 37 | 690 | 750 | 75 | 0 | 0 |
| Fraktion 38 | 430 | 520 | 50 | 0 | 0 |
| Fraktion 39 | 400 | 550 | 50 | 0 | 0 |
| Fraktion 40 | 350 | 600 | 50 | 0 | 0 |
| Fraktion 41 | 450 | 975 | 75 | 0 | 0 |
| Fraktion 42 | 375 | 1050 | 75 | 0 | 0 |
| Fraktion 43 | 200 | 750 | 50 | 0 | 0 |
| Fraktion 44 | 175 | 750 | 75 | 0 | 0 |
| Fraktion 45 | 150 | 750 | 100 | 0 | 0 |
| Fraktion 46 | 75 | 825 | 100 | 0 | 0 |
| Fraktion 47 | 0 | 900 | 100 | 0 | 0 |
| Fraktion 48 | 0 | 750 | 250 | 0 | 0 |
| Fraktion 49 | 0 | 1050 | 450 | 0 | 0 |
| Fraktion 50 | 0 | 900 | 600 | 0 | 0 |
| Fraktion 51 | 0 | 750 | 750 | 0 | 0 |
| Fraktion 52 | 0 | 250 | 750 | 0 | 0 |
| Fraktion 53 | 0 | 375 | 1050 | 75 | 0 |
| Fraktion 54 | 0 | 250 | 600 | 150 | 0 |
| Fraktion 55 | 0 | 250 | 500 | 250 | 0 |
| Fraktion 56 | 0 | 150 | 500 | 350 | 0 |
| Fraktion 57 | 0 | 50 | 500 | 450 | 0 |
| Fraktion 58 | 0 | 50 | 500 | 500 | 0 |
| Fraktion 59 | 0 | 50 | 500 | 500 | 0 |
| Fraktion 60 | 0 | 125 | 525 | 750 | 0 |
| Fraktion 61 | 0 | 50 | 350 | 600 | 0 |
| Fraktion 62 | 0 | 0 | 350 | 650 | 0 |
| Fraktion 63 | 0 | 0 | 250 | 750 | 0 |
| Fraktion 64 | 0 | 0 | 200 | 800 | 0 |
| Fraktion 65 | 0 | 0 | 150 | 850 | 0 |
| Fraktion 66 | 0 | 0 | 100 | 900 | 0 |
| Fraktion 67 | 0 | 0 | 0 | 1000 | 0 |
| Fraktion 68 | 0 | 0 | 0 | 1000 | 0 |
| Fraktion 69 | 0 | 0 | 0 | 1000 | 0 |
| Fraktion 70 | 0 | 0 | 0 | 1000 | 0 |
| Fraktion 71 | 0 | 0 | 0 | 1000 | 0 |
| Fraktion 72 | 0 | 0 | 0 | 1000 | 0 |
| Fraktion 73 | 0 | 0 | 0 | 0 | 1000 |

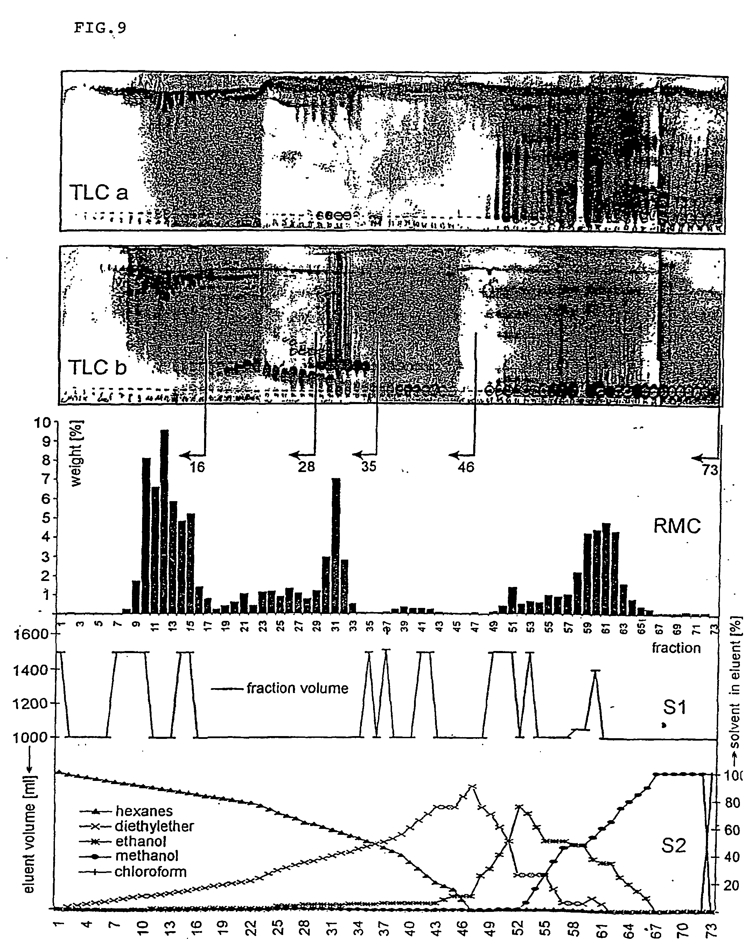

Ein Anteil von 50 µl aller Fraktionen wurde auf jeweils zwei Dünnschichtchromatographieplatten aufgetragen. Die Dünnschichtchromato-graphie-Platten waren vorgefertigte, vorbeschichtete Silica-Platten der Größe 10*20cm (Merck, Darmstadt, Deutschland). Als Fließmittel wurde Chloroform/Methanol/Wasser (70:29:1) bei der einen Platte, und reines Chloroform bei der anderen Platte angewendet. Entwickelt wurden die Platten mit dem Anisaldehyd-Schwefelsäure-Reagenz nach DAB. Das Elutionsschema, die relativen Gewichte der einzelnen Fraktionen sowie die Dünnschichtchromatographiekarten zeigt Figur 9.
Die 73 Fraktionen wurden nach dünnschichtchromatographischen Gesichtspunkten wieder zu Sammelfraktionen vereinigt. Es wurden die Sammelfraktionen 1 bis 16, 17 bis 28, 29 bis 35, 36 bis 46, und 47 bis 73 gebildet. Diese Einteilung ist in Figur 9 angegeben. Aus jeder Fraktion wurden Aliquote von je 2% (V/V) zur Durchführung der Tierversuche sowie der Gaschromatographie/Massenspek-trometrie abgenommen.

### (v) Gaschromatographie-Massenspektrometrie (GC-MS); Etablierung einer Methode zum Spurennachweis der Prostaglandine A₂, E₂, F₂ₐ und I₂ (Prostacyclin)

Die Vergleichs-Prostaglandine waren: Prostaglandin A₂ ([5Z, 13E, 15S]-15-Hydroxy-9-oxoprosta-5,10,13-trien-1-oic acid), E₂ ([5Z, 11a, 13E, 15S]-11,15-dihydroxy-9-oxoprosta-5,13-dien-1-oic acid) und F₂ₐ ([5Z, 9 a, 13E, 15S]-9,11,15-trihydroxyprosta-5,13-dien-1-oic acid) der Firma Sigma, St. Louis (MO, U.S.A.); Prostacyclin (syn. Prostaglandin I₂, [5Z, 9 a, 11a, 13E, 15S]-6,9-Epoxy-11,15-dihydroxyprosta-5,13-dien-1-oic acid) der Firma ICN Biomedicals Inc., Eschwege (Deutschland).

Zur Derivatisierung der Reinsubstanzen sowie der Proben als TMS-Ether wurde mit einer Mischung aus BSTFA und TMCS in Pyridin nach dem Protokoll des Herstellers (Fa. Macherey & Nagel, Düren, Germany) vorgegangen. Typischerweise wurden 1 mg Vergleichssubstanz bzw. die zur Trockne eingeengten Aliquote der Einzelfraktionen (ca. 1-5 mg) mit 90 ml BSTFA, 30 ml TMCS und 30 ml Pyridin versetzt und in einem fest versiegelten Mikrovial 30 Minuten auf 120°C erhitzt. Im Falle der Reinsubstanzen wurden von den so erhaltenen Stammlösungen (6.7 mg/ml) für die GC Verdünnungen in den Verhältnissen 1:20 (333 ng/ml) und 1:100 (67 ng/ml) unter Einsatz von BSTFA Reagenzlösung als Diluens hergestellt. Es zeigte sich dabei, dass die Abnahme des Gesamtionenflusses (TIC) der Chromatogramme der Verringerung der Konzentrationen der Substanzen in den Lösungen direkt proportional war.

Als Instrument kam ein GC-Q der Fa. Finnigan MAT, Bremen (Germany) mit einer GC-Säule vom Typ DB-5 zum Einsatz. Das GC-Temperaturprogramm startete bei 100°C mit einer Haltezeit von einer Minute, erhöhte bei einer Aufheizrate von 8°C/Minute bis auf 280°C und hielt diese Endtemperatur für 20 Minuten. Das Injektionsvolumen betrug 1 ml mit Split (1:20) bei den Vergleichssubstanzen bzw. ohne Split bei den Analysenproben. Die MS-Bedingungen waren wie folgt: Beschleunigungsspannung von 70 keV, Messung im positiven EI-Modus im Bereich m/z 50 bis 1000 bei 3 Microscans, Quellentemperatur 200°C, Temperatur der Transferline 220°C.

Anhand des Beispiels von Prostaglandin E₂ wurde die Anzahl der Spülzyklen der Injektionsvorrichtung ermittelt, die notwendig waren, um die Substanzen in solchen Messungen, die denjenigen der Messung der Vergleichssubstanzen direkt folgen, sicher nicht mehr nachweisen zu können. Dieser Nachweis konnte nach Injektion des unverdünnten Silylierungsansätzen von Prostaglandin E₂ nach drei regulären Spülzyklen mit Dichlormethan gerade noch erbracht werden. Daraufhin wurde die Anzahl der Spülzyklen nach jeder Injektion auf jeweils fünf festgelegt. Darüber hinaus erfolgten alle weiteren Messungen von Vergleichssubstanzen mit den mindestens 1:20 verdünnten Lösungen, so dass die Möglichkeit der Kreuzkontamination ausgeschlossen war.

Bei einer GC-Gesamtlaufzeit von 43,5 Minuten wurden unter Berücksichtigung einer Ausblendung des Lösungsmittels (4 Minuten) auf diese Weise MS-Chromatogramme mit einer zeitlichen Auflösung von 0,99 Sekunden erhalten. Die mittlere Schwankung der Retentionszeiten der Vergleichssubstanzen betrug 1,0 Sekunde, entsprach also genau einem Massen-Scan. Für die Vergleichssubstanzen wurden folgende Retentionszeiten bestimmt: Prostaglandin A₂ 20,37 Minuten, Prostaglandin E₂ 21,51 Minuten, Prostaglandin F₂ₐ 21,32 Minuten, Prostaglandin I₂ (Prostacyclin) 22,06 Minuten. Die nachfolgende Tabelle 1 stellt die charakteristischen, oberhalb von m/z 200 registrierten Massenbruchstücke zusammen, die für die selektive Suche nach Prostaglandinen durch Erstellung von Einzel-Massenspuren der GC-Chromatogramme, jeweils bezogen auf eine der charakteristischen Massen, herangezogen wurden. Die Effizienz dieser Vorgehensweise ergibt sich aus folgenden Tatsachen: Es bestehen einerseits eine auffällige Übereinstimmung der Fragmentbildung (→ universelle Massenspuren bei z.B. m/z 227, 317, 388) bei den Prostaglandinen A₂, E₂ und I₂, andererseits aber auch gewisse Analogien der Fragmentbildung beim Prostaglandin F₂ₐ. Entsprechend erfolgte die neben der Anlage der vorgenannten universellen Massenspuren eine Erfassung mit gesteigerter Toleranzbreite [→ "range"] bei m/z 462/463 und 552/553, sowie unter Einbeziehung zusätzlicher Einzelmassen (→ m/z 391, 537) die gezielte Erfassung typischer Bruchstücke von Prostaglandin F₂ₐ.

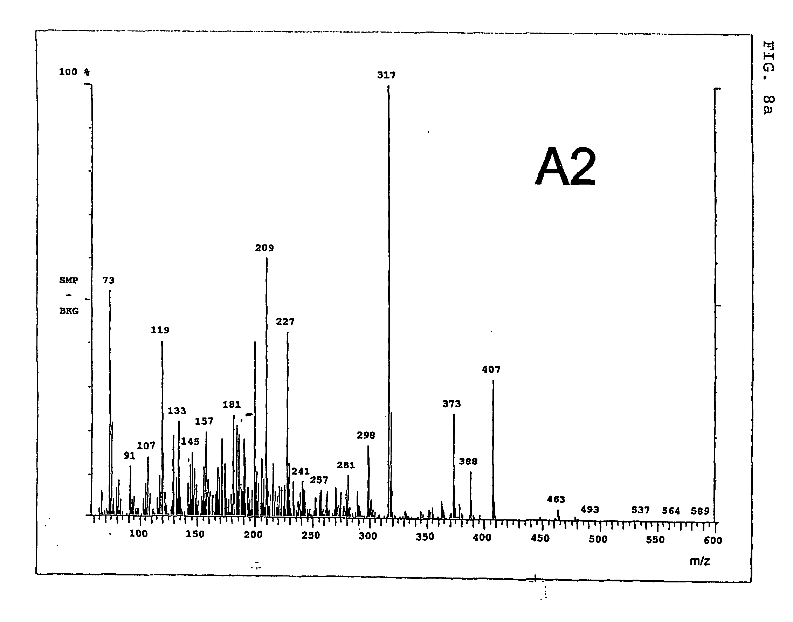

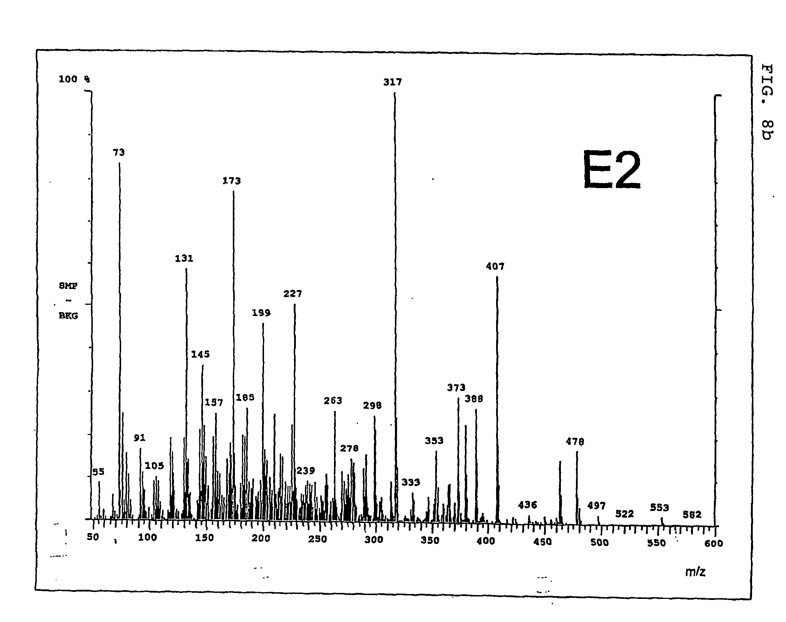

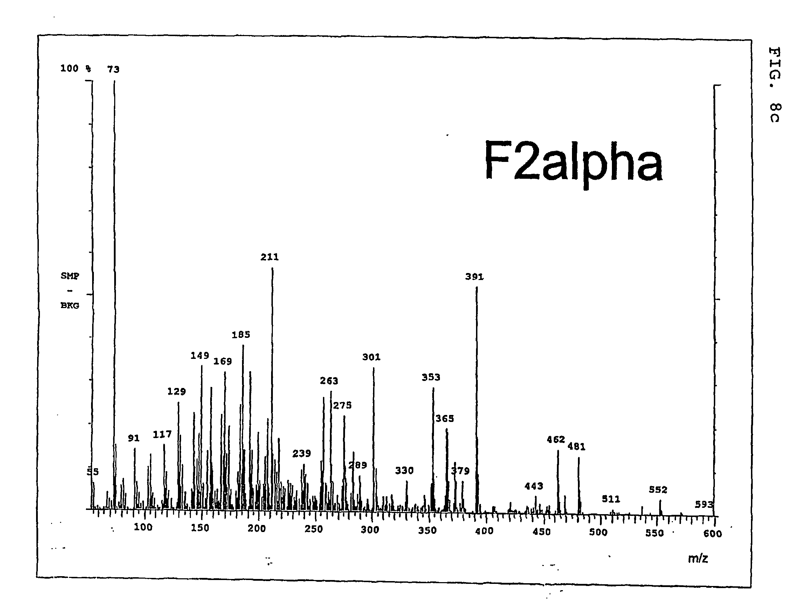

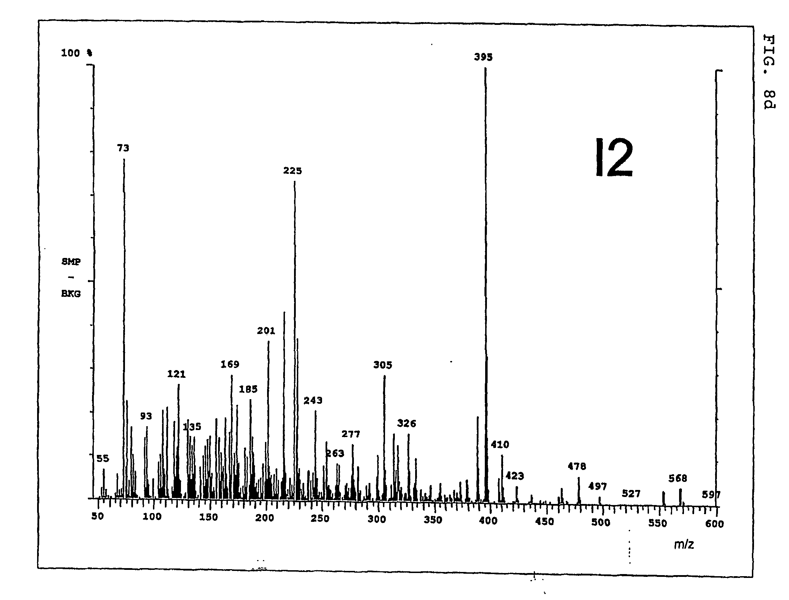

Die zugehörigen Massenspektren zeigen die Figuren 8a bis 8d.

**Tabelle 1**

| Charakteristische Massenbruchstücke der TMS-ether der untersuchten reinen Prostaglandine A₂, E₂, F₂ₐ und I₂. | | | |
|---|---|---|---|
| Prostaglandin | t_{R} [min.] | M⁺ [m/z] | typical fragmentary ions [m/z] above 200 au |
| A₂ | 20,37 | 478 | 227, 317, 373, 388, 407, 463 |
| E₂ | 21,51 | 568 | 227, 317, 373, 388, 391, 407, 463, 478 |
| F₂ₐ | 21,32 | 642 | 211, 301, 353, 372, 391, 462, 481, 537, 552 |
| I₂ | 22,06 | 568 | 227, 317, 388, 395, 463, 478, 553 |

### (vi) Nachweis der verschiedenen Prostaglandine MS in den Testfraktionen mittels Gaschromatographie-Massenspektrometrie (GC-MS)

Die Konzentration der verdünnten Referenzlösungen für die Prostaglandine betrug 333 ng/ml (1:20) und 67 ng/ml (1:100); die Konzentrationen der aus den Aliquoten der 73 Einzelfraktionen hergestellten TMS-Derivatisierungsansätze betrug jeweils ca. 6700-20000 ng/ml. Unter Einbeziehung des dynamischen Meßbereiches der GC-MS Messungen errechnet sich damit die Nachweisgrenze der Prostaglandine in den Fraktionen auf 2.2 ppm. Die dieser Berechnung zugrundegelegte Dynamik der massenspektrometrischen Identifizierung nach Trennung war besser als 1:40; sie wurde berechnet als Relation der dem Ionenstrom proportionalen, minimal notwendigen Fläche eines Peaks im Massenchromatogramm, mit der ein solches Massenspektrums erzielbar war, das im Spektrenvergleich noch sicher als identisch mit dem authentischen Vergleichsspektrum erkannt wird. Umgerechnet kann ein Tier im erfindungsgemäßen Versuch von einem Prostaglandin nicht mehr als ~0.6⁻¹⁰ M erhalten haben können. Diese Menge ist in Bezug auf den Blutdruck irrelevant und erklärt den hier gezeigten Effekt nicht.

### (vii) Zubereitung der Proben für die Tierversuche

Zur Herstellung der Proben für die Tierversuche wurden wässrige Suspensionen der fettartigen Rückstände in andelsüblichem *Aqua ad injectabilia* unter Zuhilfenahme von Ethylalkohol hergestellt. Dazu wurde der fettartige Rückstand zunächst mit dem Ethanol benetzt und mit dem Wasser im Verhältnis 1:19 (V/V) aufgefüllt. Dieses Gemisch wurde dann mit Hilfe eines handelsüblichen Ultraschall Mikrosuspendiergerätes ein- bis dreimal für je 15 bis 30 Sekunden bei 5 bis 30 Watt Ausgangsleistung homogenisiert.

### (viii) Ermittlung der mittleren Teilchengröße und der Größenverteilung der injizierten Suspensionen mittels Photon Correlation Spectroscopy (PCS).

Die Bestimmung der Teilchengrößenverteilung in den durch Mikrosuspension hergestellten wässrigen Suspensionen erfolgte mit Hilfe der Photon Correlation Spectoscopy (PCS). Dazu wurde ein Autosizer der Firma Malvern Instruments (Malvern Autosizer 2c series 7032, Herrsching, Germany), ausgerüstet mit einem Multi-8 Correlator, verwendet. Die turbidimetrisch ermittelten Werte für die Größe der kolloidalen Partikel werden jeweils als Mittelwerte und Standardabweichungen von fünf getrennten Messungen angegeben. Die Photonen-korrelations-spektroskopisch bestimmte Turbidität des injizierbaren Kolloids des Extrakts, das wie oben beschrieben zubereitet worden war, wurde durch Tröpfchen mit einer Größe von 143 ± 47 nm verursacht. Das Kolloid, das aus der Fraktion 47 bis 73 zubereitet worden war, hatte eine mittlere Tröpfchengröße von 188 ± 12 nm. Die Partikel hatten somit eine Tröpfchengröße, die unter der Toleranzgrenze der Lungengängigkeit lag, und war somit für den Versuch galenisch einwandfrei.

### (ix) Tierversuche

Für die Tierversuche wurden adulte, spontan hypertensive Wistar-Ratten mit einem durchschnittlichen Gewicht von etwa 400g verwendet. In einer Ketanest/Diazepam-Narkose wurden die Einzeldosen (10mg/kg KG) von einem Volumen von 1500 µl innerhalb von 10 Sekunden über die *Vena jugularis* in die *Vena cava superior* injiziert. Die Blutdruckmessung erfolgte intraarteriell in der *Arteria iliaca communis.* Es wurde ein Druckwandler in Verbindung mit einem Siemens Sirecust 404 Monitor verwendet. Zur Eichung der Druckwerte wurde ein Quecksilbermanometer nach *Gauer* verwendet. Die Aufzeichnung des Blutdruckes erfolgte analog auf einem handelsüblichen Schreiber sowie digital. Der Beobachtungszeitraum betrug jeweils eine Stunde. In einer Gruppe von sieben Tieren betrug der Blutdruckabfall nach der Dosis von 10 mg/Kg Körpergewicht des Extraktes vor der Chromatographie: 20,71 ± 5,37/17,71 ± 6,72 mmHg nach zwei Minuten, 29,57 ± 16,61/24,85 ± 14,69 mmHg nach einer Stunde. Dieser Unterschied war mit einem p=0.0078 statistisch signifikant.

In einer Gruppe von sieben Tieren betrug der Blutdruckabfall nach einer Dosis von 10 mg/Kg Körpergewicht der Fraktion 47-73: 37,57 ± 22,14/28,57 ± 16,99 mmHg nach 2 Minuten, und 57,42 ± 29,07/45,57 ± 22,41 mmHg nach einer Stunde. Dieser Unterschied war mit einem p=0.0078 statistisch signifikant.

Somit hatten sowohl der Extrakt, als auch die wiedervereinigte Fraktion 47-73 den im oben beschriebenen Effekt.

### 2. Blutdrucksteigernde Aktivität (Medullopressin)

Zur Gewinnung der blutdrucksteigernden Aktivität wurde wie oben unter Punkt 1. Material und Methoden (i) bis (iv) im Zusammenhang mit der Gewinnung des biutdrucksenkenden Aktivität beschrieben vorgegangen.

### (x) Gelchromatographie

Die beiden dort (vgl. Punkt iv) zuletzt genannten Fraktionen wurden in einer Gelchromatographie auf Sephadex LH-20 (Fluka, Buchs, Schweiz) weiter getrennt. Hierzu wurde bei Fraktion 36 bis 46 eine 143 cm hoch gefüllte Säule mit einem Innendurchmesser von 4,2 cm und einer Füllmenge von etwa 500 g verwendet; bei Fraktion 47 bis 73 handelte es sich um eine 95 cm hoch gefüllte Säule mit einem Innendurchmesser von 6,3 cm und eine Füllmenge von etwa 700 g. Eluiert wurde jeweils mit einem Gemisch aus Methanol und Dichlormethan. Eluiert wurde jeweils mit einem Gemisch aus Methanol und Dichlormethan (50/50), (V/V). Die Aktivität fand sich in den Fraktionen 1 bis 276, erhalten aus der Fraktion 47 bis 73 der Kieselgelsäule; die Fraktionsgröße betrug jeweils 12 ml. Als Vergleichsmaterial wurden die korrespondierenden Fraktionen, die sich ebenfalls früh eluierenden Fraktionen 1 bis 48, aus der Fraktion 36 bis 46 herangezogen.

### (xi) Zubereitung der Proben für die Tierversuche

Zur Herstellung der Proben für die Tierversuche wurden wässrige Suspensionen der fettartigen Rückstände in handelsüblichem *Aqua ad injectabilia* unter Zuhilfenahme von Ethylalkohol (96%) hergestellt. Dazu wurde der fettartige Rückstand zunächst mit dem Ethanol benetzt und mit dem Wasser im Verhältnis 1:19 (V/V) aufgefüllt. Dieses Gemisch wurde dann mit Hilfe eines Ultraschall-Mikrosuspendiergerätes einbis dreimal für je 15 bis 30 Sekunden bei 5 bis 30 Watt Ausgangsleistung homogenisiert. Als Lösemittelkontrolle wurde ein Gemisch aus Ethanol/Wasser 5/95 (V/V) benutzt.

### (xii) Dosierung der Gemische und angewendete Gemische

Es wurden die obengenannten Sammelfraktionen 1 bis 276, in der sich die hier beschriebene blutdruckerhöhende Aktivität fand, zum Vergleich die Sammelfraktion 1 bis 48, ebenfalls wie oben beschrieben, und die beschriebene Lösemittelkontrolle an jeweils sieben Ratten untersucht. Das injizierte Volumen von 1,5 ml wurde jeweils über etwa 10 Sekunden gespritzt. Die Dosierung betrug jeweils 10 mg/kg Körpergewicht.

### (xiii) Tierversuche

Für die Tierversuche wurden 21 adulte, spontan hypertensive Wistar-Ratten (SHR/N Crl BR) mit einem durchschnittlichen Gewicht von etwa 400 g verwendet. In einer Ketanest-Diazepamnarkose (Ketanest 50mg/kg Kg; Diazepam 3 mg/kg Kg) wurden die Einzeldosen von einem Volumen von 800µl innerhalb von 15 Sekunden über die Vena jugularis in die *Vena cava superior* injiziert. Diese Dosierung entsprach jeweils 50 mg/kg KG, Lee et al (Lee, o.a.O.) folgend. Die Blutdruckmessung erfolgte intraarteriell in der *Arteria iliaca communis* bzw. in der distalen Aorta, je nach Lage des Katheters, von einem Zugang an der rechten *Arteria femoralis* aus. Es wurde ein handelsüblicher Druckwandler in Verbindung mit einem Sirecust 404 Monitor verwendet. Zur Eichung der mit dieser Einheit zu messenden Druckwerte wurde ein Quecksilbermanometer nach *Gauer* verwendet. Die Aufzeichnung des Blutdruckes erfolgte analog auf einem handelsüblichen Schreiber Kompensograph III, sowie digital über einen handelsüblichen Personalcomputer bei einer Sample-Frequenz von 17Hz. Der Beobachtungszeitraum betrug jeweils eine Stunde.

### (xiv) Statistik

Zur statistischen Auswertung wurde zunächst die Latenz der Zeit bis zum Höhepunkt des Blutdruckanstiegs nach der Gabe des Gemisches Fraktion 1 bis 276 ermittelt. Sodann wurden die Blutdruckwerte dieses Zeitpunktes jeweils mit den Blutdruckwerten unmittelbar vor der Gabe der Gemische verglichen. Hierzu wurde der U-Test von Whitney und Mann, unter Zuhilfenahme des Programms Graph Pad Prism (Firma Graph Pad, USA) verwendet. Ein signifikanter Unterschied wurde bei einem p<0.05 angenommen.

### (xv) Ergebnisse der Tierversuche

1.) Fraktion 1 bis 276. In dieser Fraktion fand sich bei Injektion eine starke blutdruck erhöhende Aktivität, die 55 ± 27 Sekunden nach Beendigung der Injektion ihren Höhepunkt erreichte, und 201 ± 59 Sekunden andauerte. Es ist zu sehen, dass fast unmittelbar nach Beginn der Injektion der Blutdruck kontinuierlich ansteigt, einen Höhepunkt erreicht, und dann etwas langsamer wieder auf das Ausgangsniveau absinkt. Der Anstieg betrug systolisch 30,1 ± 7,1 mmHg und diastolisch 34,7 ± 6 mmHg (p=0,0003).
2.) Fraktion 1 bis 48 (Vergleichsfraktion). Bei Injektion dieser Fraktion fand sich keine wesentliche Änderung des Blutdrucks. Es ist keine wesentliche Beeinflussung des Blutdruckes zu beobachten. Zum Zeitpunkt des Blutdruckhöhepunktes nach der Gabe der Fraktionen 1 bis 276, also 55 Sekunden nach dem Ende der Injektion, war systolisch ein leichter Abfall des Blutdruckes um 1,1 ± 3 mmHg, und diastolisch ein leichter Abfall um 0,3 ± 5,1 mmHg zu verzeichnen. Diese Unterschiede waren jeweils statistisch nicht signifikant.
3.) Lösemittelkontrolle: Bei der Injektion eines Gemisches von Ethanol/Wasser, wie es zur Suspendierung der oben beschriebenen Fraktionen verwendet worden war, fand sich ebenfalls keine wesentliche Änderung des Blutdruckes bei diesen Ratten. Zum Zeitpunkt des Blutdruckhöhepunktes nach der Gabe der Fraktionen 1 bis 276, also 55 Sekunden nach dem Ende der Injektion, war systolisch ein leichter Abfall des Blutdruckes um 0,1 ± 3 mmHg, und diastolisch ein leichter Abfall um 0,9 ± 2,2 mmHg zu verzeichnen. Diese Unterschiede waren jeweils statistisch ebenfalls nicht signifikant.

Eine solche Aktivität, wie hier beschreiben, wurde auch bei Injektion anderer Suspensionen fettiger Fraktionen nicht gefunden.

## Patentansprüche

1. Verfahren zur Isolierung einer blutdrucksenkenden Aktivität aus dem Nierenmark von Säugetieren durch
(i) Trocknung des Säugetiernierenmarks,
(ii) Extraktion des getrockneten Nierenmarks mit einem organischen Lösungsmittel mit einer Dielektrizitätskonstante zwischen 2 und 45,
(iii) Chromatographie des erhaltenen Extraktes über ein hydrophiles Sorbens mit einem Eluens-Gradienten von unpolar zu polar, und
(iv) Gewinnung der blutdrucksenkenden Aktivität als Extrakt nach Abtrennung des Elutionsmittels.

2. Verfahren nach Anspruch 1, **dadurch gekennzeichnet, dass** die Säugetiere ausgewählt sind aus der Gruppe bestehend aus Schweinen, Kaninchen, Ratten, Schafen, Hunden, Rindern und Primaten.

3. Verfahren nach Anspruch 1, **dadurch gekennzeichnet, dass** das organische Lösungsmittel ausgewählt ist aus einem C₁-C₃-Chlorkohlenwasserstoff oder überkritischem Kohlendioxid.

4. Verfahren nach Anspruch 3, **dadurch gekennzeichnet, dass** der C₁-C₃-Chlorkohlenwasserstoff ausgewählt ist aus der Gruppe bestehend aus Chloroform, Methylendichlorid, 1,1-Dichlorethan, 1,2-Dichlorethylen und 1,1,2-Trichlorethan.

5. Verfahren nach Anspruch 1, **dadurch gekennzeichnet, dass** das hydrophile Sorbens ausgewählt ist aus der Gruppe bestehend aus regulären und irregulären Kieselgelen.

6. Verfahren nach Anspruch 5, **dadurch gekennzeichnet, dass** das Kieselgel eine Korngröße zwischen 3 und 500 µm und eine Porengröße zwischen 4 und 12 nm (40 bis 120 Å) ausweist.

7. Verfahren nach Anspruch 1, **dadurch gekennzeichnet, dass** der bei der Chromatographie verwendete Eluens-Gradient besteht aus C₅-C₁₀-Alkan, C₅-C₁₀-Alkan/C₂-C₈-Ether/Ethanol, C₂-C₈-Ether/Ethanol, C₂-C₈-Ether/Ethanol/Methanol, Ethanol/Methanol, Methanol und Chloroform.

8. Verfahren nach Anspruch 5, **dadurch gekennzeichnet, dass** das C₅-C₁₀-Alkan ausgewählt ist aus der Gruppe bestehend aus, n-Pentan, n-Hexan, cylco-Hexan, n-Heptan, n- oder iso-Oktan, n- oder iso-Nonan, n-und iso-Dekan.

9. Verfahren nach Anpruch 8, **dadurch gekennzeichnet, dass** der C₂-C₈-Ether ausgewählt ist aus der Gruppe bestehend aus Diethylether, Diisopropylether, Ethylisopropylether, tert-Butylmethylether, Tetrahydrofuran und Dioxan.

10. Blutdrucksenkendes Extrakt erhältlich nach dem Verfahren wie in irgendeinem der Ansprüche 1 bis 9 definiert.

11. Pharmazeutische Zusammensetzung umfassend das blutdrucksenkende Extrakt gemäß Anspruch 10.

12. Verwendung des blutdrucksenkenden Extrakts gemäß Anspruch 10 oder der pharmazeutischen Zusammensetzung gemäß Anspruch 11 zur Herstellung eines Medikaments zur Senkung des Blutdrucks bei Säugetieren.

13. Verfahren zur Isolierung einer blutdruckerhöhenden Aktivität aus dem Nierenmark von Säugetieren durch
(i) Trocknung des Säugetiernierenmarks,
(ii) Extraktion des getrockneten Nierenmarks mit einem organischen Lösungsmittel mit einer Dielektrizitätskonstante zwischen 2 und 45,
(iii) Chromatographie des erhaltenen Extraktes über ein hydrophiles Sorbens mit einem Eluens-Gradienten von unpolar zu polar,
(iv) Gewinnung der blutdrucksenkenden Aktivität als Extrakt nach Abtrennung des Elutionsmittels und
(v) Gewinnung der blutdruckerhöhenden Aktivität durch Gelchromatographie des Rückstandes mit einem organischen Lösungsmittel mit einer Dielektrizitätskonstante zwischen 2 und 45.

14. Verfahren nach Anspruch 13, **dadurch gekennzeichnet, dass** die Säugetiere ausgewählt sind aus der Gruppe bestehend aus Schweinen, Kaninchen, Ratten, Schafen, Hunden, Rindern und Primaten.

15. Verfahren nach Anspruch 13, **dadurch gekennzeichnet, dass** das organische Lösungsmittel in Schritt (ii) ausgewählt ist aus einem C₁-C₃-Chlorkohlenwasserstoff oder überkritischem Kohlendioxid.

16. Verfahren nach Anspruch 15, **dadurch gekennzeichnet, dass** der C₁-C₃-Chlorkohlenwasserstoff ausgewählt ist aus der Gruppe bestehend aus Chloroform, Methylendichlorid, 1,1-Dichlorethan, 1,2-Dichlorethylen und 1,1,2-Trichlorethan.

17. Verfahren nach Anspruch 13, **dadurch gekennzeichnet, dass** das hydrophile Sorbens ausgewählt ist aus der Gruppe bestehend aus regulären und irregulären Kieselgelen.

18. Verfahren nach Anspruch 17, **dadurch gekennzeichnet, dass** das Kieselgel eine Korngröße zwischen 3 und 500 µm und eine Porengröße zwischen 4 und 12 nm (40 bis 120 Å) ausweist.

19. Verfahren nach Anspruch 13, **dadurch gekennzeichnet, dass** der bei der Chromatographie verwendete Eluens-Gradient besteht aus C₅-C₁₀-Alkan, C₅-C₁₀-Alkan/C₂-C₈-Ether/Ethahol, C₂-C₈-Ether/Ethanol, C₂-C₈-Ether/Ethanol/Methanol, Ethanol/Methanol, Methanol und Chloroform.

20. Verfahren nach Anspruch 19, **dadurch gekennzeichnet, dass** das C₅-C₁₀-Alkan ausgewählt ist aus der Gruppe bestehend aus, n-Pentan, n-Hexan, cyclo-Hexan, n-Heptan, n- oder iso-Oktan, n- oder iso-Nonan, n-und iso-Dekan.

21. Verfahren nach Anpruch 19, **dadurch gekennzeichnet, dass** der C₂-C₈-Ether ausgewählt ist aus der Gruppe bestehend aus Diethylether, Diisopropylether, Ethylisopropylether, tert.-Butylmethylether, Tetrahydrofuran und Dioxan.

22. Verfahren nach Anspruch 13, **dadurch gekennzeichnet, dass** das organische Lösungsmittel bei der Gelchromatographie in Schritt (v) ausgewählt ist aus der Gruppe bestehend aus C₁-C₄-Alkoholen, C₂-C₈-Ethern, C₅-C₁₀ aliphatischen Alkanen, C₆-C₁₂ aromatischen Kohlenwasserstoffen, C₃-C₉ Ketonen und deren Gemischen.

23. Blutdruckerhöhendes Extrakt erhältlich nach dem Verfahren wie in irgendeinem der Ansprüche 13 bis 22 definiert.

24. Pharmazeutische Zusammensetzung umfassend das blutdruckerhöhende Extrakt gemäß Anspruch 23.

25. Verwendung des blutdruckerhöhenden Extrakts gemäß Anspruch 23 oder der pharmazeutischen Zusammensetzung gemäß Anspruch 24 zur Herstellung eines Medikaments zur Erhöhung des Blutdrucks bei Säugetieren.

## Claims

1. A method for isolating a hypotensive activity from the renal medulla of mammals by
(i) drying the mammal renal medulla;
(ii) extracting the dried renal medulla with an organic solvent having a dielectric constant of between 2 and 45;
(iii) subjecting the extract obtained to chromatography over a hydrophilic sorbent using an eluent gradient of from non-polar to polar; and
(iv) recovering the hypotensive activity as an extract after separating off the eluent.

2. The method according to claim 1, **characterized in that** said mammals are selected from the group consisting of pigs, rabbits, rats, sheep, dogs, bovines and primates.

3. The method according to claim 1, **characterized in that** said organic solvent is selected from a C₁-C₃ chlorohydrocarbon or supercritical carbon dioxide.

4. The method according to claim 3, **characterized in that** said C₁-C₃ chlorohydrocarbon is selected from the group consisting of chloroform, methylene dichloride, 1,1-dichloroethane, 1,2-dichloroethylene and 1,1,2-trichloroethane.

5. The method according to claim 1, **characterized in that** said hydrophilic sorbent is selected from the group consisting of regular and irregular silica gels.

6. The method according to claim 5, **characterized in that** said silica gel has a grain size of between 3 and 500 µm and a pore size of between 4 and 12 nm (40 to 120 Å).

7. The method according to claim 1, **characterized in that** the eluent gradient used in said chromatography consists of C₅-C₁₀ alkane, C₅-C₁₀ alkane/C₂-C₈ ether/ethanol, C₂-C₈ ether/ethanol, C₂-C₈ ether/ethanol/methanol, ethanol/methanol, methanol, and chloroform.

8. The method according to claim 5, **characterized in that** said C₅-C₁₀ alkane is selected from the group consisting of n-pentane, n-hexane, cyclohexane, n-heptane, n- or iso-octane, n- or iso-nonane, n- and iso-decane.

9. The method according to claim 8, **characterized in that** said C₂-C₈ ether is selected from the group consisting of diethyl ether, diisopropyl ether, ethyl isopropyl ether, tert-butyl methyl ether, tetrahydrofuran and dioxan.

10. A hypotensive extract obtainable by the method as defined in any of claims 1 to 9.

11. A pharmaceutical composition comprising the hypotensive extract according to claim 10.

12. Use of the hypotensive extract according to claim 10 or of the pharmaceutical composition according to claim 11 for preparing a medicament for lowering the blood pressure in mammals.

13. A method for isolating a hypertensive activity from the renal medulla of mammals by
(i) drying the mammal renal medulla;
(ii) extracting the dried renal medulla with an organic solvent having a dielectric constant of between 2 and 45;
(iii) subjecting the extract obtained to chromatography over a hydrophilic sorbent using an eluent gradient of from non-polar to polar;
(iv) recovering the hypotensive activity as an extract after separating off the eluent; and
(v) recovering the hypertensive activity by subjecting the residue to gel chromatography with an organic solvent having a dielectric constant of between 2 and 45.

14. The method according to claim 13, **characterized in that** said mammals are selected from the group consisting of pigs, rabbits, rats, sheep, dogs, bovines and primates.

15. The method according to claim 13, **characterized in that** said organic solvent in step (ii) is selected from a C₁-C₃ chlorohydrocarbon or supercritical carbon dioxide.

16. The method according to claim 15, **characterized in that** said C₁-C₃ chlorohydrocarbon is selected from the group consisting of chloroform, methylene dichloride, 1,1-dichloroethane, 1,2-dichloroethylene and 1,1,2-trichloroethane.

17. The method according to claim 13, **characterized in that** said hydrophilic sorbent is selected from the group consisting of regular and irregular silica gels.

18. The method according to claim 17, **characterized in that** said silica gel has a grain size of between 3 and 500 µm and a pore size of between 4 and 12 nm (40 to 120 Å).

19. The method according to claim 13, **characterized in that** the eluent gradient used in said chromatography consists of C₅-C₁₀ alkane, C₅-C₁₀ alkane/C₂-C₈ ether/ethanol, C₂-C₈ ether/ethanol, C₂-C₈ ether/ethanol/methanol, ethanol/methanol, methanol, and chloroform.

20. The method according to claim 19, **characterized in that** said C₅-C₁₀ alkane is selected from the group consisting of n-pentane, n-hexane, cyclohexane, n-heptane, n- or iso-octane, n- or iso-nonane, n- and iso-decane.

21. The method according to claim 19, **characterized in that** said C₂-C₈ ether is selected from the group consisting of diethyl ether, diisopropyl ether, ethyl isopropyl ether, tert-butyl methyl ether, tetrahydrofuran and dioxan.

22. The method according to claim 13, **characterized in that** said organic solvent used in the gel chromatography in step (v) is selected from the group consisting of C₁-C₄ alcohols, C₂-C₈ ethers, C₅-C₁₀ aliphatic alkanes, C₆-C₁₂ aromatic hydrocarbons, C₃-C₉ ketones and their mixtures.

23. A hypertensive extract obtainable by the method as defined in any of claims 13 to 22.

24. A pharmaceutical composition comprising the hypertensive extract according to claim 23.

25. Use of the hypertensive extract according to claim 23 or of the pharmaceutical composition according to claim 24 for preparing a medicament for raising the blood pressure in mammals.

## Revendications

1. Procédé d'isolement d'une activité d'abaissement de la pression sanguine depuis la substance médullaire rénale de mammifères, par
(i) séchage de la substance médullaire rénale,
(ii) extraction de la substance médullaire rénale séchée avec un solvant organique avec une constante diélectrique allant de 2 à 45,
(iii) chromatographie de l'extrait obtenu sur un sorbant hydrophile avec un gradient d'éluant allant de non polaire à polaire, et
(iv) obtention de l'activité d'abaissement de la pression sanguine comme extrait après séparation de l'éluant.

2. Procédé selon la revendication 1, **caractérisé en ce que** les mammifères sont choisis parmi le groupe consistant en les porcs, les lapins, les rats, les moutons, les chiens, les boeufs et les primates.

3. Procédé selon la revendication 1, **caractérisé en ce que** le solvant organique est choisi parmi un hydrocarbure chloré en C₁-C₃ ou le dioxyde de carbone supercritique.

4. Procédé selon la revendication 3, **caractérisé en ce que** l'hydrocarbure chloré en C₁-C₃ est choisi parmi le groupe consistant en le chloroforme, le dichlorure de méthylène, le 1,1-dichloroéthane, le 1,2-dichloroéthylène et le 1,1,2-trichloroéthane.

5. Procédé selon la revendication 1, **caractérisé en ce que** le sorbant hydrophile est choisi parmi le groupe consistant en les gels de silice réguliers et irréguliers.

6. Procédé selon la revendication 5, **caractérisé en ce que** le gel de silice présente une granulométrie allant de 3 à 500 µm et une taille des pores allant de 4 à 12 nm (40 à 120 Å).

7. Procédé selon la revendication 1, **caractérisé en ce que** le gradient d'éluant utilisé pour la chromatographie consiste en un alcane en C₅-C₁₀, alcane en C₅-C₁₀/éther C₂-C₈/éthanol, éther C₂-C₈/éthanol, éther C₂-C₈/éthanol/méthanol, éthanol/méthanol, méthanol et chloroforme.

8. Procédé selon la revendication 7, **caractérisé en ce que** l'alcane en C₅-C₁₀ est choisi parmi le groupe consistant en le n-pentane, le n-hexane, le cyclohexane, le n-heptane, le n- ou l'isooctane, le n-ou l'isononane, le n- et l'isodécane.

9. Procédé selon la revendication 8, **caractérisé en ce que** l'éther C₂-C₈ est choisi parmi le groupe consistant en le diéthyléther, le diisopropyléther, l'éthylisopropyléther, le t-butylméthyléther, le tétrahydrofuranne et le dioxanne.

10. Extrait abaissant la pression sanguine, pouvant être obtenu par le procédé comme défini dans l'une des revendications 1 à 9.

11. Composition pharmaceutique comprenant l'extrait abaissant la pression sanguine selon la revendication 10.

12. Utilisation de l'extrait abaissant la pression sanguine selon la revendication 10 ou de la composition pharmaceutique selon la revendication 11, pour la préparation d'un médicament pour abaisser la pression sanguine chez un mammifère.

13. Procédé d'isolement d'une activité d'élévation de la pression sanguine depuis la substance médullaire rénale de mammifères, par
(i) séchage de la substance médullaire rénale,
(ii) extraction de la substance médullaire rénale séchée avec un solvant organique avec une constante diélectrique allant de 2 à 45,
(iii) chromatographie de l'extrait obtenu sur un sorbant hydrophile avec un gradient d'éluant allant de non polaire à polaire, et
(iv) obtention de l'activité d'abaissement de la pression sanguine comme extrait après séparation de l'éluant, et
(v) obtention de l'activité d'élévation de la pression sanguine par chromatographie sur gel du résidu avec un solvant organique ayant une constante diélectrique allant de 2 à 45.

14. Procédé selon la revendication 13, **caractérisé en ce que** les mammifères sont choisis parmi le groupe consistant en les porcs, les lapins, les rats, les moutons, les chiens, les boeufs et les primates.

15. Procédé selon la revendication 13, **caractérisé en ce que** le solvant organique de l'étape (ii) est choisi parmi un hydrocarbure chloré en C₁-C₃ ou le dioxyde de carbone supercritique.

16. Procédé selon la revendication 15, **caractérisé en ce que** l'hydrocarbure chloré en C₁-C₃ est choisi parmi le groupe consistant en le chloroforme, le dichlorure de méthylène, le 1,1-dichloroéthane, le 1,2-dichloroéthylène et le 1,1,2-trichloroéthane.

17. Procédé selon la revendication 13, **caractérisé en ce que** le sorbant hydrophile est choisi parmi le groupe consistant en les gels de silice réguliers et irréguliers.

18. Procédé selon la revendication 17, **caractérisé en ce que** le gel de silice présente une granulométrie allant de 3 à 500 µm et une taille des pores allant de 4 à 12 nm (40 à 120 Å).

19. Procédé selon la revendication 13, **caractérisé en ce que** le gradient d'éluant utilisé pour la chromatographie consiste en un alcane en C₅-C₁₀, alcane en C₅-C₁₀/éther C₂-C₈/éthanol, éther C₂-C₈/éthanol, éther C₂-C₈/éthanol/méthanol, éthanol/méthanol, méthanol et chloroforme.

20. Procédé selon la revendication 19, **caractérisé en ce que** l'alcane en C₅-C₁₀ est choisi parmi le groupe consistant en le n-pentane, le n-hexane, le cyclohexane, le n-heptane, le n- ou l'isooctane, le n- ou l'isononane, le n- et l'isodécane.

21. Procédé selon la revendication 19, **caractérisé en ce que** l'éther C₂-C₈ est choisi parmi le groupe consistant en le diéthyléther, le diisopropyléther, l'éthylisopropyléther, le t-butylméthyléther, le tétrahydrofuranne et le dioxanne.

22. Procédé selon la revendication 13, **caractérisé en ce que** le solvant organique pour la chromatographie sur gel de l'étape (v) est choisi parmi le groupe consistant en des alcools en C₁-C₄, des éthers C₂-C₈, des alcanes aliphatiques en C₅-C₁₀, des hydrocarbures aromatiques en C₆-C₁₂, des cétones en C₃-C₉ et leurs mélanges.

23. Extrait élevant la pression sanguine, pouvant être obtenu par le procédé comme défini dans l'une des revendications 13 à 22.

24. Composition pharmaceutique comprenant l'extrait élevant la pression sanguine selon la revendication 23.

25. Utilisation de l'extrait élevant la pression sanguine selon la revendication 23 ou de la composition pharmaceutique selon la revendication 24, pour la préparation d'un médicament pour élever la pression sanguine chez un mammifère.
